# EUROPEAN PATENT SPECIFICATION

(11) **EP 1 562 927 B1**
(45) Date of publication and mention of the grant of the patent: **13.05.2009**
(21) Application number: 03757580.0
(22) Date of filing: 09.10.2003
(51) Int. Cl.: C07D 305/14, A61K 31/335, C07D 205/08, C07C 271/22, C07C 227/22

(54) **THIO-ANALOGUES OF PACLITAXEL AND INTERMEDIATES THEREOF**
THIO-ANALOGA VON PACLITAXEL UND DEREN VORPRODUKTE
NOUVEAUX TAXANES, LEURS MODES D'UTILISATION ET LEURS PROCEDES DE PREPARATION

(30) Priority: 09.10.2002 US 417270 P
(43) Date of publication of application: 17.08.2005
(73) Proprietor: Chatham Biotec Ltd., Riverview NB E1B 3W1 (CA)
(72) Inventor: NAIDU, Ragina, Burnaby, British Columbia V5G 2P2 (CA)
(74) Representative: Walker, Ross Thomson
(86) International application number: PCT/CA2003/001521
(87) International publication number: WO 2004/033442

(56) References cited:
- WO-A-94/13655
- WO-A-94/29288
- US-A- 5 821 363
- US-A1- 2001 014 746
- HART D J ET AL: "PREPARATION OF PRIMARY AMINES AND 2-AZETIDINONES VIA NU- TRIMETHYLSILYL IMINES" JOURNAL OF ORGANIC CHEMISTRY, AMERICAN CHEMICAL SOCIETY. EASTON, US, vol. 48, no. 3, 11 February 1983 (1983-02-11), pages 289-294, XP002064429 ISSN: 0022-3263
- HATA ET AL.: "Reaction of aziridinecarboxylic acids with thiols in aqueous solution. the formation of beta-amino acids" TETRAHEDRON, vol. 43, no. 17, 1987, pages 3881-3888, XP002273416
- YOSHITERU HATA ET AL: "REACTION OF AZIRIDINECARBOXYLIC ACIDS WITH THIOLS IN AQUEOUS SOLUTION. THE FORMATION OF BETA-AMINO ACID" TETRAHEDRON, ELSEVIER SCIENCE PUBLISHERS, AMSTERDAM, NL, vol. 43, no. 17, 1987, pages 3881-3888, XP001179911 ISSN: 0040-4020
- AGGARWAL ET AL.: "Additions of stabilised and semi-stabilised sulfur ylides to tosyl protected imines: are they under kinetic or thermodynamic control?" J. CHEM. SOC, PERKIN TRANS., vol. 1, 2001, pages 3159-3166, XP002290269
- LEE ET AL.: ORG. LETT., vol. 2, no. 9, 2000, pages 1243-1246, XP002290270
- CROUSSE ET AL.: "First Stereoselective Synthesis of cis 3-CF3-Aziridine-2-caroxylates. A Route to New (Trifluoromethyl) alpha-Functionalised beta-Amino Acids" SYNLETT, no. 5, 2001, pages 679-681, XP001182594
- VAN DER VEEN ET AL.: "Synthesis of Azetindine-2,3-diones (alpha-Keto beta-Lactams)via 3-(Phenylthio)-2-azetidinones" J. ORG. CHEM., vol. 54, no. 24, 1989, pages 5758-5762, XP002290271
- MADAN ET AL.: "A new synthetic approach for novel C-3-substituted beta-lactams" TETRAHEDRON LETTERS, vol. 41, 2000, pages 5577-5581, XP002290272
- MANHAS ET AL.: "A CONVENIENT SYNTHESIS OF AZETIDINE-2,3-DIONES (ALPHA-KETO-BETA-LASCTAMS)" TETRAHEDRON LETTERS, vol. 25, no. 42, 1984, pages 4733-4736, XP002290273
- HART ET AL.: "Prpeparation of Primary Amines and 2-Azetidinones via N-Trimethylsilyl Imines" J. ORG. CHEM., vol. 48, no. 3, 1983, pages 289-294, XP002290274
- FREIHAMMER ET AL.: "Halogenation of 4-Phenyl-3-(phenylsulfonyl)-2-azetidinones with N-Halosuccinimides. Kinetic vs. Thermodynamic Control" J. ORG. CHEM., vol. 65, no. 21, 2000, pages 7203-7207, XP002290275

## Description

### Field of the Invention

The present invention relates generally to taxanes, compounds useful in the preparation of taxanes, and synthetic methods useful in the preparation of taxanes.

### Description of the Related Art

The taxane family of terpenes has received much attention in the scientific and medical community because members of this family have demonstrated broad spectrum anti-leukemic and tumor-inhibitory activity. A well-known member of this family is paclitaxel, which has the following structure, 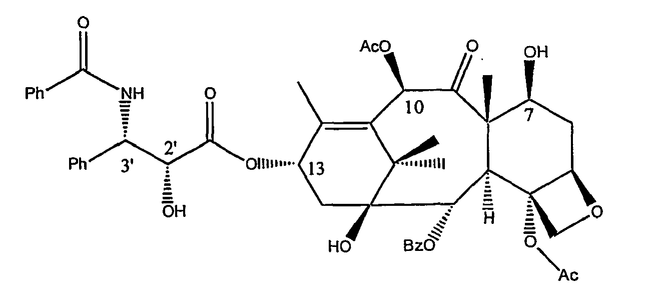 wherein Ac is acetyl, Bz is benzoyl, Ph is phenyl, the 2' position has the R configuration and the 3' position has the S configuration. Paclitaxel was first isolated from the bark of the pacific yew tree (*Taxus brevifolia*) in 1971, and has proved to be a potent natural anticancer agent. For example, paclitaxel has been found to have activity against different forms of leukemia and against solid tumors in the breast, ovary, brain, and lung in humans.

This activity has stimulated an intense research effort over recent years, including the search for other taxanes having similar or improved properties, and the development of synthetic pathways for making taxanes such as paclitaxel. One result from this research effort was the discovery of an analog of paclitaxel called taxotere. Taxotere has been found to have very good anti-tumor activity and better bio-availability than paclitaxel. Taxotere is similar in structure to paclitaxel, having t-butoxycarbonyl instead of benzoyl on the amino group at the 3' position, and a hydroxyl group instead of the acetoxy group at the C-10 position (see EP 253738 for a discussion of taxotere).

Taxanes are structurally complicated molecules, and the development of commercially viable synthetic methods to make taxanes has been a challenge. Semi-synthetic pathways have been developed, where these methods begin with the isolation of a naturally occurring material and then the conversion of that material to the taxane of interest. One such pathway for the semi-synthesis of paclitaxel begins with 10-deacetylbaccatin III, a taxane isolated from the needles of the English yew tree (*Taxus baccata*)*.* A semi-synthetic route for the production of taxotere has been reported that involve coupling of N-tert-butoxycarbonyl-(2R, 3S)-3-phenylisoserine with 10-deacetylbaccatin III in conjunction with proper protecting groups (Tetrahedron Letters 33:5185, 1992). The synthesis of taxotere has also been reported using enantiomerically pure beta-lactams as intermediates (J. Org. Chem. 56:1681, 1991; Tetrahedron 48:6985, 992).

Synthesis of beta-lactam from an aldehyde via N-trimethylsilyl imine intermediates is described by Hart et al. (J. Org. Chem. 48:289, 1983).

U.S. Applicator Publication No. US2001/0014746 by Holton discloses a process for preparing N-acyl, N-sulfonyl and N-phosporyl substituted isoserine esters in which a metal alkoxide is reacted with a beta-lactam. Holton further discloses a process of preparing a N-substituted beta-lactam via a N-substituted imine.

Hata et al. discloses a process of preparing beta-lactam from thiol substituted unnatural amino acids (Tetrahedron 43:3881, 1987).

Aggarwal et al. discloses a process for preparing aziridines from a stabilized sulfur ylide and a tosyl protected imine (Chem. Soc. Perkin Trans. 1:3159, 2001).

Lee et al. discloses a process of preparing alpha-substituted-beta-amino ester via ring-opening of oxazollne-5-carboxylates (Org. Lett. 2(9):1243, 2000).

Crousse et al. discloses a process of preparing alpha-substituted-beta-amino ester via ring-opening of aziridine-2-carbonxylates (Synlett 5:679, 2001).

Van der Veen et al, discloses a process of preparing alpha-keto-beta-lactam via phenylthio substituted beta-lactam (J. Org. Chem. 54:5758, 1989).

Madan et al. discloses a process of chlorination of beta-lactam via a S_{N}2 substitution (Tetrahedron Lett. 41:5577, 2000).

Manhas et al. discloses a process of preparing alpha-keto-beta-lactam via a hydrolysis of 3-thiophenyl-3-chloro-beta-lactam (Tetrahedron Lett. 25(42):4733, 1984).

Freihammer et al. discloses N-protection of 3-phenylsulfonyl-beta-lactam (J. Org. Chem. 65:1203, 2000).

WO 94/29288 (Kelly et al.) discloses 7-deoxy taxol derivatives obtained by coupling of a baccatin compound and an oxazolidine free acid.

US Patent No. 5,821,363 (Wicnienski et al.) discloses 7-deoxy taxol derivatives obtained by coupling of a baccatin compound and an oxazolidine free acid.

WO 94/13655 (Hester et al.) 7-devxy taxol derivatives obtained by coupling of a baccatin compound and an oxazolidine free acid.

While significant advances have been made in this field, there remain a need for improved synthetic techniques for the production of paclitaxel and analogs thereof such as taxotere. For example, existing semi-synthetic pathways for production of paclitaxel generally involve coupling of a suitable side chain precursor to the free hydroxyl group at position 13 of 10-deacetylbaccatin III. Fully synthetic pathways also employ addition of such side-chains in a similar way. Thus, there is a need for improved routes for the generation of such precursors of the C-13 side chain, particularly since this side-chain has been found to be an important structural feature. The present invention fulfils these needs and provides other related advantages.

### BRIEF SUMMARY OF THE INVENTION

In one aspect, the present invention provides a process of preparing a beta-lactam, where the process comprises the scheme 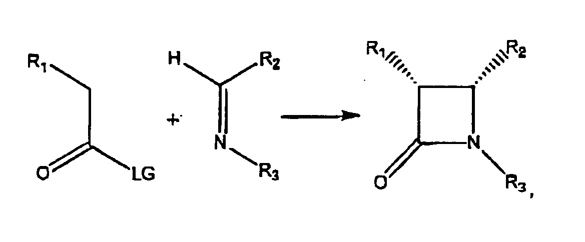 wherein R₁ is thiol, or protected thiol; LG is a leaving group; R₂ is alkyl, alkenyl, alkynyl, or aryl where R₂ is optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and R₃ is hydrogen. Optionally, (R₂)(H)C=N-R₃ is prepared by reaction between an aldehyde of the formula R₂₇-CHO, and an amine of the formula R₃-NH₂. Also optionally, R₁ is thiophenyl and R₂ is phenyl.

In another aspect, the present invention provides a compound of the formula 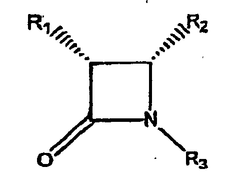 wherein R₁ is thiol (SH) or thiophenyl, R₂ is phenyl and R₃ is hydrogen, and salts thereof.

In another aspect, the present invention provides a process of opening a beta-lactam ring, where the process comprises the scheme 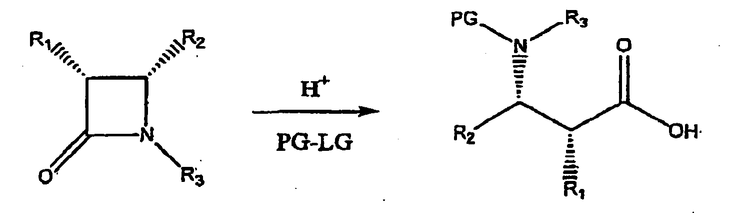 wherein R₁ is thiol, or protected thiol; LG. is a leaving group; PG is an amine protecting group; R₂ is alkyl, alkenyl, alkynyl, or aryl where R₂ is optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; R₃ is hydrogen, C₁-C₆ alkyl or aryl where R₃ is optionally substituted with one or more halogens, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and H⁺ is a proton source.

In another aspect, the present invention provides an isoserine compound of the formula 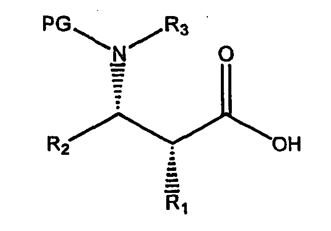 wherein R₁ is thiol, or protected thiol; PG is an amino protecting group; R₂ is alkyl, alkenyl, alkynyl, or aryl where R₂ is optionally substituted with one or more of halogen, hydroxyl, alkoxy, aryloxy, heteroaryloxy, amino, alkylamino, dialkylamino, mercapto, alkylthio, arylthio, heteroarylthio, cyan, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; R₃ is hydrogen, C₁-C₆ alkyl or aryl where R₃ is optionally substituted with one or more halogens, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and salts and esters thereof.

In another aspect, the present invention provides a process for preparing a beta lactam, comprising the scheme 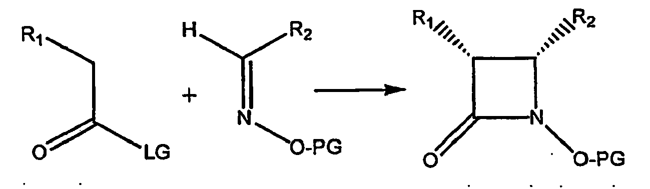 wherein R₁ is thiol, or protected thiol; LG is a leaving group; R₂ is alkyl, alkenyl, alkynyl or aryl, where R₂ may be optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and PG is a protecting group.

In another aspect, the present invention provides a compound of the formula 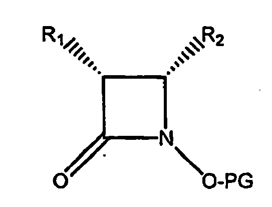 wherein R₁ is thiol, or protected thiol; R₂ is alkyl, alkenyl, alkynyl or aryl, where R₂ may be optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and PG is a protecting group.

In another aspect, the present invention provides a process comprising the scheme 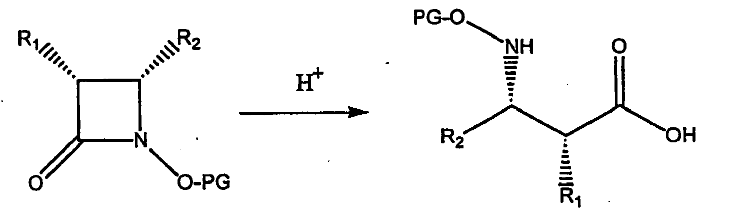 wherein R₁ is thiol, or protected thiol; R₂ is alkyl, alkenyl, alkynyl or aryl, where R₂ may be optionally substituted with one or more of halogen, hydroxyl, alkoxy, aryloxy, heteroaryloxy, amino, alkylamino, dialkylamino, mercapto, alkylthio, arylthio, heteroarylthio, cyan, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and PG is a protecting group, where H+ represents a proton source such as organic or mineral acid.

In another aspect, the present invention provides a process comprising esterifying a compound of the formula 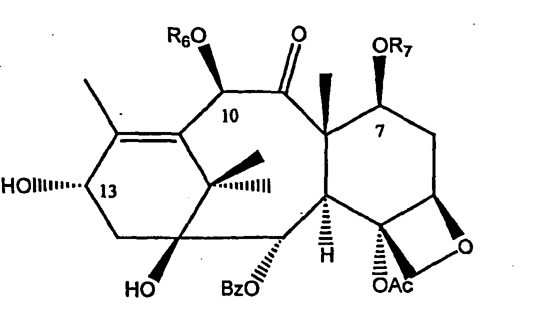 wherein R₆ is acetyl or dichloroacetyl; and R₇ is triethylsilyl, dichloroacetyl or 2,2,2-trichloroethoxycarbonyl (Troc); with an acid compound of a formula selected from 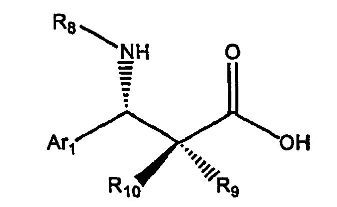 wherein R₈ is tBOC, PMP, Bz or H; R₉ is thiophenyl; and R₁₀ is hydrogen. Optionally, the acid compound has the formula 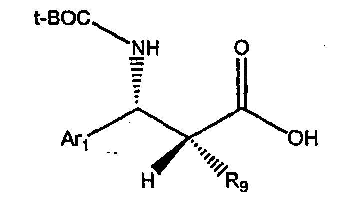 wherein Ar₁ is phenyl and R₉ is thiophenyl

In another aspect, the present invention provides a compound of the formula 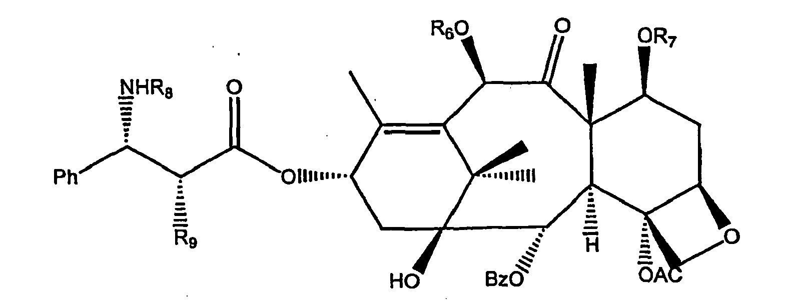 wherein R₆ and R₇ are independently selected from hydrogen, triethylsilyl, acetyl and dichloroacetyl, with the proviso that R₆ and R₇ may not be simultaneously hydrogen, R₈ is tBOC, PMP, Bz or H, and R₉ is thiophenyl, acetoxy, methoxy, t-butoxycarbonyloxy, ethoxyethyl, or dichloroacetyl. Optionally, R₆ and R₇ are each dichloroacetyl; R₈ is tBOC; and R₉ is thiophenyl. As another option, R₆ is acetyl, R₇ is TES, R₈ is t-BOC, and R₉ is thiophenyl.

In another aspect, the present invention provides a process comprising the scheme 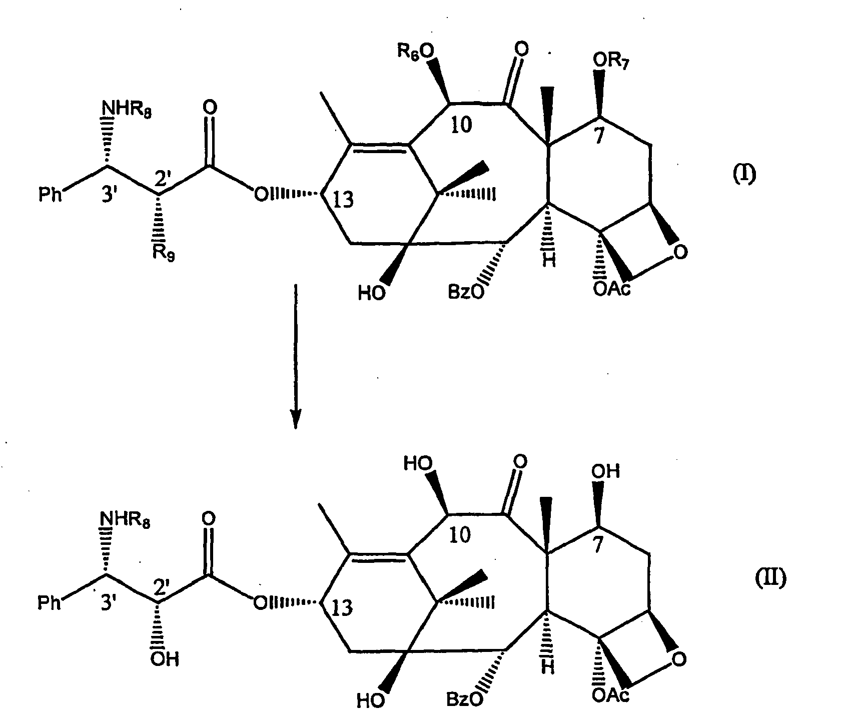 wherein R₆ and R₇ are independently selected from hydrogen, triethylsilyl, acetyl and dichloroacetyl, with the proviso that R₆ and R₇ may not be simultaneously hydrogen, R₈ is tBOC, PMP, Bz or H, and R₉ is thiophenyl. Optionally, the compound of structure (I) is deprotected at the 2' position to form an intermediate of structure (Ia), and the intermediate is treated with zinc acetate dihydrate to form the compound of formula (II), where the intermediate has the structure 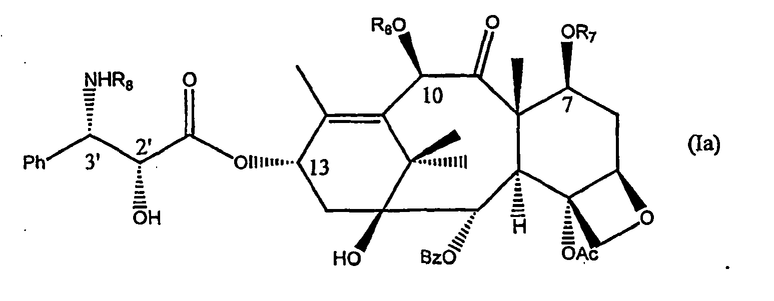 Also optionally, the compound of formula (I) is treated with protic acid and tertiary amine in an organic solvent to form an intermediate of formula (Ib), and the intermediate is deprotected at the 2' position to form the compound of formula (II), where the intermediate has the structure

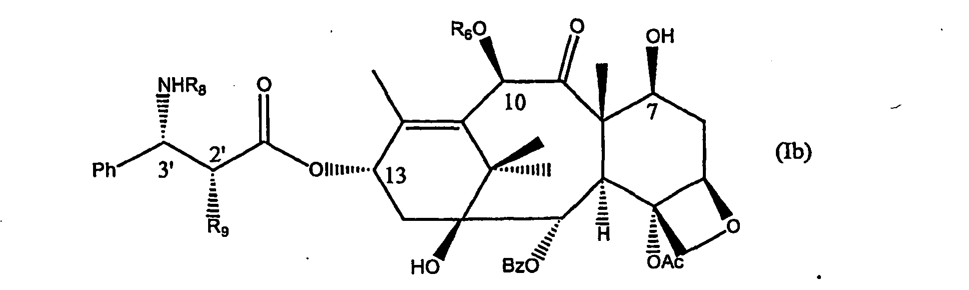

In another aspect, the present invention provides a method of preparing TAXOTERE, comprising reacting a compound of structure (III) with t-BOC, followed by deprotection of at least one of the 2', 7 and 10 positions, where the compound of structure (III) is 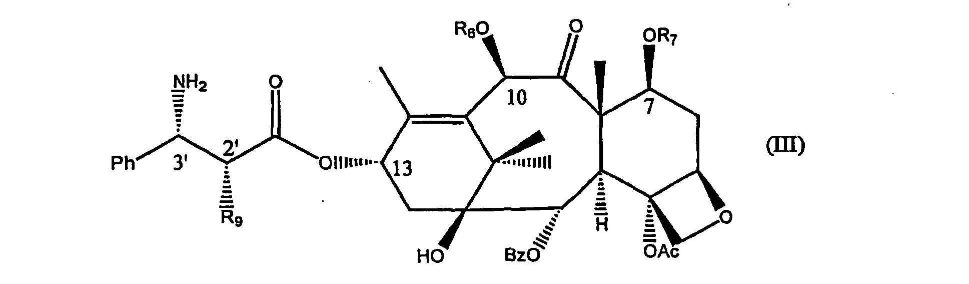 wherein R₆ and R₇ are independently selected from hydrogen, triethylsilyl, acetyl, Troc and dichloroacetyl, with the proviso that R₆ and R₇ may not be simultaneously hydrogen, and R₉ is thiophenyl.

In another aspect, the present invention provides a process of coupling a beta lactam to a baccatin III compound according to the following scheme 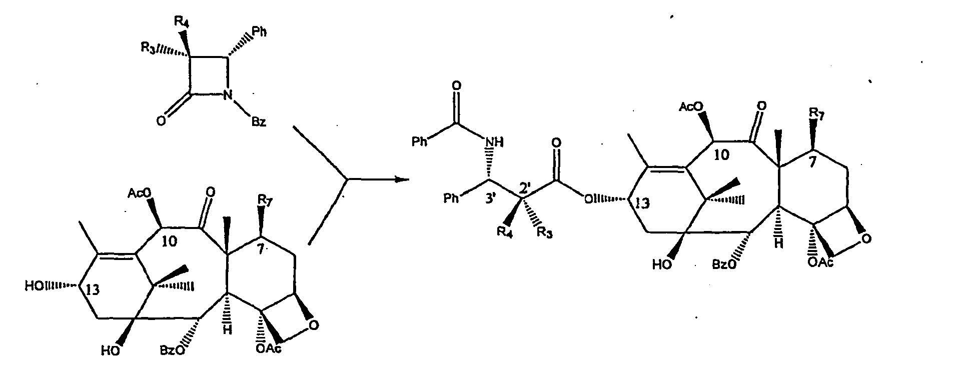 wherein; at least one of R₃ or R₄ is either thiol or protected thiol; and, the other of R₃ or R₄ is independently selected from hydrogen, hydroxyl, protected hydroxyl, thiol, protected thiol, alkyl, alkenyl, alkynyl, or aryl where R₃ and R₄ are optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; R₇ is.hydroxyl.or a.protected hydroxyl group; and the coupling is performed by addition of metal hydride, metal alkoxide or lewis acid to the reaction mixture.

In another aspect, the present invention provides a process for making a compound of formulas (III') or (IV'): 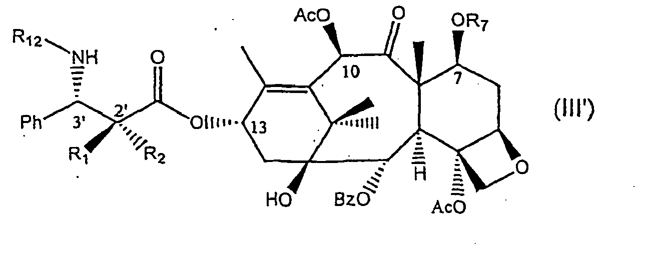 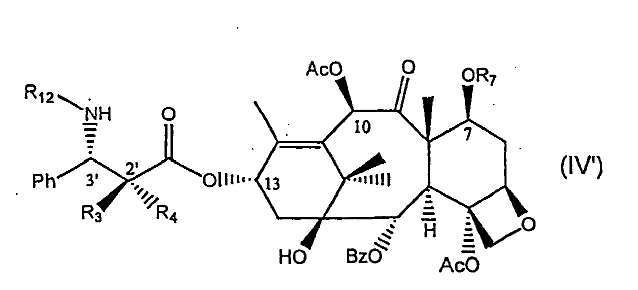 comprising the step of reacting a compound of formula (I') 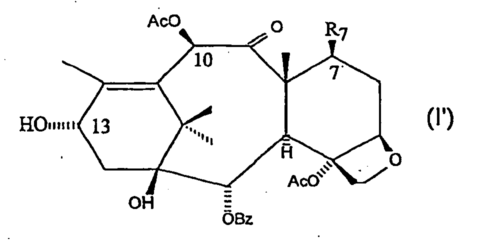 with a compound of formula (IIa') or (IIb') 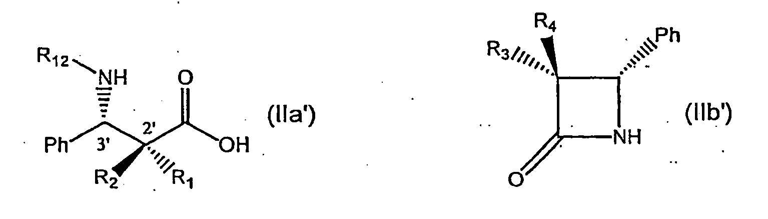 wherein; at least one of R₃ or R₄ and/or at least one of R₁ or R₂ is thiol or protected thiol; the others of R₁, R₂, R₃ and R₄ being independently selected from hydrogen, hydroxyl, protected hydroxyl, thiol, protected thiol, linear, branched or cyclic alkyl, alkenyl, alkynyl, or aryl where R₁ and R₃ are optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; R₇ = -OCOCHCl₂, triethylsilyl or Troc; and R₁₂ is an amine protecting group.

These and other aspects of this invention will be evident upon reference to the following detailed description.

### BRIEF DESCRIPTION OF THE FIGURES

Figure 1 illustrates several chemical routes for the preparation of beta-lactam and phenylisoserine sidechains according to the present invention.
Figure 2 illustrates a chemical route for the preparation of beta-lactam and phenylisoserine sidechains according to the present invention.
Figure 3 illustrates a chemical route for the preparation of a beta-lactam and phenylisoserine sidechain according to the present invention.
Figure 4 illustrates chemical routes for the preparation of taxotere from various intermediate compounds prepared according to the present invention.
Figure 5 illustrates chemical routes for the preparation of taxotere from various intermediate compound prepared according to the present invention.

### DETAILED DESCRIPTION OF THE INVENTION

In brief, the present invention relates to 3-phenylisoserine compounds as well as the preparation thereof and the intermediates formed during their preparation; baccatin III compounds and the preparation thereof; methods of joining together a 3-phenylisoserine compound and a baccatin III compound as well as the resulting chemical structure(s); and the conversion of one taxane compound to another taxane compound as well as the resulting chemical structure(s). Before providing a detailed description of these and other aspects of the present invention, the following list of definitions is provided to assist the reader in understanding the invention.

### A. Definitions

The term "hydroxy-protecting group" refers to a readily cleavable group bonded to the oxygen of a hydroxyl (-OH) group. Examples of hydroxy protecting groups include, without limitation, acetyl (Ac), benzyl (PhCH₂), 1-ethoxyethyl (EE), methoxymethyl (MOM), (methoxyethoxy)methyl (MEM), (p-methoxyphenyl)methoxymethyl (MPM), tert-butyldimethylsilyl (TBS), tert-butyldiphenylsilyl (TBPS), tert-butoxycarbonyl (tBoc, t-Boc, tBOC, t-BOC), tetrahydropyranyl (THP), triphenylmethyl (Trityl, Tr), 2-methoxy-2-methylpropyl, benzyloxycarbonyl (Cbz), trichloroacetyl (OCCCl₃), 2,2,2-trichloroethoxycarbonyl (Troc), benzyloxymethyl (BOM), tert-butyl (t-Bu), triethylsilyl (TES), trimethylsilyl (TMS), and triisopropylsilyl (TIPS). The related term "protected hydroxy group" refers to a hydroxy group that is bonded to a hydroxy-protecting group. General examples of protected hydroxy groups include, without limitation, -O-alkyl, -O-acyl, acetal, and -O-ethoxyethyl, where some specific protected hydroxy groups include, formyloxy, acetoxy, propionyloxy, chloroacetoxy, bromoacetoxy, dichloroacetoxy, trichloroacetoxy, trifluoroacetoxy, methoxyacetoxy, phenoxyacetoxy, benzoyloxy, benzoylformoxy, p-nitro benzoyloxy, ethoxycarbonyloxy, methoxycarbonyloxy, propoxycarbonyloxy, 2,2,2-trichloro ethoxycarbonyloxy, benzyloxycarbonyloxy, tert.-butoxycarbonyloxy, 1-cyclopropyl ethoxycarbonyloxy, phthaloyloxy, butyryloxy, isobutyryloxy, valeryloxy, isovaleryloxy, oxalyoxy, succinyloxy and pivaloyloxy, phenylacetoxy, phenylpropionyloxy, mesyloxy, chlorobenzoyloxy, para-nitrobenzoyloxy, para-tert-butyl benzoyloxy, capryloyloxy, acryloyloxy, methylcarbamoyloxy, phenylcarbamoyloxy, naphthylcarbamoyloxy, and the like. Hydroxy protecting groups and protected hydroxy groups are described in, *e.g.,* C. B. Reese and E. Haslam, "Protective Groups in Organic Chemistry," J. G. W. McOmie, Ed., Plenum Press, New York, N.Y., 1973, Chapters 3 and 4, respectively, and T. W. Greene and P. G. M. Wuts, "Protective Groups in Organic Synthesis," Second Edition, John Wiley and Sons, New York, N.Y., 1991, Chapters 2 and 3.

The term "thiol-protecting group" refers to a readily cleavable group bonded to the sulfur of a thiol (-SH) group. Examples of thiol protecting groups include, without limitation, triphenylmethyl (trityl, Trt), acetamidomethyl (Acm), benzamidomethyl, 1-ethoxyethyl, benzoyl, and the like. The related term "protected thiol group" refers to a thiol group that is bonded to a thiol-protecting group. General examples of protected thiol groups include, without limitation, -S-alkyl (alkylthio, e.g., C₁-C₁₀alkylthio), -S-aryl (thiophenyl), -S-acyl (acylthio), thioacetal, -S-aralkyl (aralkylthio, *e.g.,* aryl(C₁-C₄)alkylthio), where some specific protected thiols groups include methylthio, ethylthio, propylthio, isopropylthio, butylthio, isobutylthio, sec-butylthio, tert-butylthio, pentylthio, isopentylthio, neopentylthio, hexylthio, heptylthio, nonylthio, cyclobutylthio, cyclopentylthio and cyclohexylthio, benzylthio, phenethylthio, propionylthio, n-butyrylthio and iso-butyrylthio. Thio protecting groups and protected thio groups are described in, *e.g.,* C. B. Reese and E. Haslam, "Protective Groups in Organic Chemistry," J. G. W. McOmie, Ed., Plenum Press, New York, N.Y., 1973, Chapters 3 and 4, respectively, and T. W. Greene and P. G. M. Wuts, "Protective Groups in Organic Synthesis," Second Edition, John Wiley and Sons, New York, N.Y., 1991, Chapters 2 and 3.

The term "amine protecting group" refers to groups known in the art that can be used to protect an amine group from undergoing an undesired chemical reaction. Examples of amine protecting groups include, but are not limited to: acyl types such as formyl, trifluoroacetyl, phthalyl, and p-toluenesulfonyl; aromatic carbamate types such as benzyloxycarbonyl (Cbz) and substituted benzyloxy-carbonyls, 1-(p-biphenyl)-l-methylethoxy-carbonyl, and 9-fluorenylmethyloxycarbonyl (Fmoc); aliphatic carbamate types such as tert-butyloxycarbonyl (tBoc), ethoxycarbonyl, diisopropylmethoxycarbonyl, and allyloxycarbonyl; cyclic alkyl carbamate types such as cyclopentyloxycarbonyl and adamantyloxycarbonyl; alkyl types such as triphenylmethyl and benzyl; trialkylsilane such as trimethylsilane; and thiol containing types such as phenylthiocarbonyl and dithiasuccinoyl. Amine protecting groups and protected amine groups are described in, *e.g*., C. B. Reese and E. Haslam, "Protective Groups in Organic Chemistry," J. G. W. McOmie, Ed., Plenum Press, New York, N.Y., 1973, Chapters 3 and 4, respectively, and T. W. Greene and P. G. M. Wuts, "Protective Groups in Organic Synthesis," Second Edition, John Wiley and Sons, New York, N.Y., 1991, Chapters 2 and 3.

The following Table shows the chemical structure of some protecting groups, as well as nomenclature used to identify those chemical structures.

| | | | |
|---|---|---|---|
| Acetyl (Ac) | 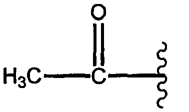 | Acetoxy (-OAc) | 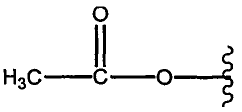 |
| Dichloroacetyl | 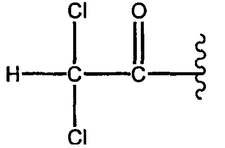 | Dichloroacetoxy | 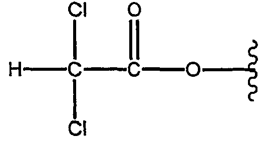 |
| Triethylsilyl (TES) | 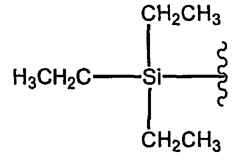 | Triethylsiloxy (-OTES) | 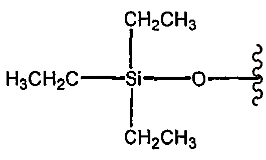 |
| Benzoyl | 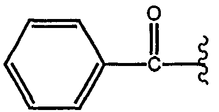 | Benzoyloxy | 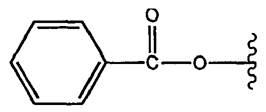 |
| t-Butyloxycarbonyl (tBOC) | | 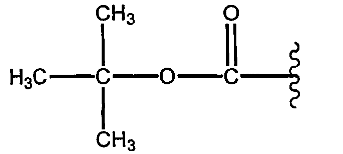 | |
| t-Butoxycarbonyloxy (-O-tBOC) | | 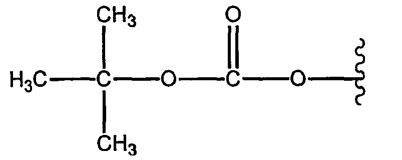 | |
| para-Methoxyphenyl (PMP) | | 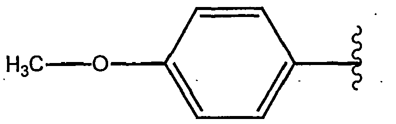 | |

The term "alkyl" refers to a hydrocarbon structure wherein the carbons are arranged in a linear or branched manner.

Lower alkyl refers to alkyl groups of from 1 to 5 carbon atoms. Examples of lower alkyl groups include methyl, ethyl, propyl, isopropyl, butyl, s- and t-butyl and the like. Preferred alkyl groups are those of C20 or below. More preferred alkyl groups are those of C13 or below. Cycloalkyl includes cyclic hydrocarbon groups of from 3 to 13 carbon atoms. Examples of cycloalkyl groups include cyclopropyl, cyclobutyl, cyclopentyl, norbornyl, adamantyl and the like. When an alkyl residue having a specific number of carbons is named, all geometric isomers having that number of carbons are intended to be encompassed; thus, for example, "butyl" is meant to include n-butyl, sec-butyl, isobutyl and t-butyl; "propyl" includes n-propyl and isopropyl.

The term "alkenyl" refers to an alkyl group having at least one site of unsaturation, i.e., at least one double bond.

The term "alkynyl" refers to an alkyl group having at least one triple bond between adjacent carbon atoms.

The terms "alkoxy" and "alkoxyl" both refer to moieties of the formula -O-alkyl. Examples include methoxy, ethoxy, propoxy, isopropoxy, cyclopropyloxy, cyclohexyloxy and the like. Lower-alkoxy refers to groups containing one to four carbons. The analogous term "aryloxy" refers to moieties of the formula -O-aryl.

The term "acyl" refers to moieties of the formula -C(=O)-alkyl. One or more carbons in the acyl residue may be replaced by nitrogen, oxygen or sulfur as long as the point of attachment to the parent remains at the carbonyl. Examples include acetyl, benzoyl, propionyl, isobutyryl, t-butoxycarbonyl, benzyloxycarbonyl and the like. Lower-acyl refers to groups containing one to four carbons.

The term aryl refers to phenyl or naphthyl. Substituted aryl refers to mono- and poly- substituted phenyl or naphthyl. Exemplary subsituents for aryl include one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms.

The term "heteroaryl" refers to a 5- or 6-membered heteroaromatic ring containing 1-3 heteroatoms selected from O, N, or S; a bicyclic 9- or 10-membered heteroaromatic ring system containing 0-3 heteroatoms selected from O, N, or S; or a tricyclic 13- or 14-membered heteroaromatic ring system containing 0-3 heteroatoms selected from O, N, or S. Exemplary aromatic heterocyclic rings include, e.g., imidazole, pyridine, indole, thiophene, benzopyranone, thiazole, furan, benzimidazole, quinoline, isoquinoline, quinoxaline, pyrimidine, pyrazine, tetrazole and pyrazole.

The term "leaving group" (LG) refer to a chemical moiety that may be displaced during a substitution or elimination reaction. Exemplary leaving groups include halide (*e.g*., bromide and chloride) and as tosyl.

The term "halogenating agent" refers to a chemical that may be added to a reaction mixture to cause the addition of a halide to a carbon of an organic molecule. Halogenating agents include, for example, inorganic acid halides, for example thionyl chloride, phosphorus trichloride, phosphorus tribromide, phosphoryl chloride trifluoromethanesulfonic acid, N-iodosuccinimide and phosphorus pentachloride. Other halogenating are known in the art. The reaction is conveniently carried out in the presence of an excess of the halogenating agent in the presence of a solvent or diluent such as, for example, a halogenated solvent such as methylene chloride, chloroform or carbon tetrachloride. The reaction may conveniently carried out at a temperature in the range, for example, 10 to 150°C, preferably in the range 40 to 100°C.

In several instances, the present invention provides compounds including the designation "-CO₂-E where E represents hydrogen or an organic group. In these instances, the compounds being disclosed are carboxylic acids or esters thereof. Optionally, E is hydrogen. Alternatively, E is an organic group, where preferred organic groups are alkyl, alkenyl, alkynyl, aryl, or heteroaryl as defined above. Optionally, E has a molecular weight of less than 1,000, preferably less than 500 g/mol.

### B. Sidechain Preparation

In various aspects, the present invention provides for the preparation of imine compounds, the conversion of an imine compound to a β-lactam compound, the preparation of oxime compounds, the conversion of an oxime compound to a β-lactam, the conversion of one β-lactam compound to another β-lactam compound, the ring-opening of a β-lactam compound to provide a 3-phenylisoserine compound, and the conversion of one 3-phenylisoserine compound to another 3-phenylisoserine compound. These various aspects of the invention are described in detail below. The individual reaction steps, the starting materials and products when novel, and sequences of reaction steps are all aspects of the present invention.

### 1. Preparation of imine compounds

In one aspect of the invention, as illustrated in Reaction 1, the reaction of benzaldehyde with anisidine yields a para-methoxyphenyl (PMP)-protected imine. 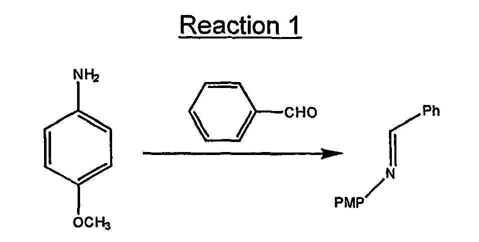 More specifically, to a solution of benzaldehyde in an inert solvent such as dichloromethane is added anisidine at about room temperature followed by magnesium sulfate and the reaction mixture stirred at room temperature for about 16 hours. The solid is filtered and the filtrate is evaporated to give the product imine.

In another aspect, the present invention provides a process of forming a beta lactam of the formula 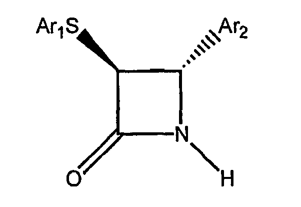 wherein Ar₁ and Ar₂ are each aryl groups, where each of Ar₁ and Ar₂ are independently optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbon atoms, and aryloxycarbonyl where the aryloxy portion contains 6 to 20 carbon atoms. The process comprises reacting together compounds of the formula Ar₁S-CH₂-C(=O)Cl, NH₃, and Ar₂-CHO under conditions that form the beta lactam. In one embodiment, each of Ar₁ and Ar₂ are phenyl.

For example, an aspect of the present invention is illustrated by Reaction 2, wherein an imine may be prepared by reacting benzaldehyde with ammonia. 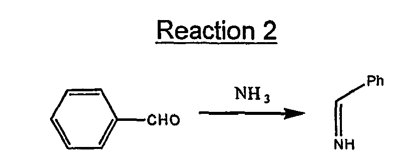 More specifically, to a solution of benzaldehyde in a suitable solvent such as ethanol is added ammonia solution at room temperature, and the stirred reaction mixture is heated to about 40-50°C for about 2-3 hours. The resulting solid is filtered and washed with methanol or equivalent followed by water to give the imine.

### 2. Conversion of an imine compound to a beta-lactam compound

In one aspect, the present invention provides a process of preparing a beta-lactam, comprising the scheme 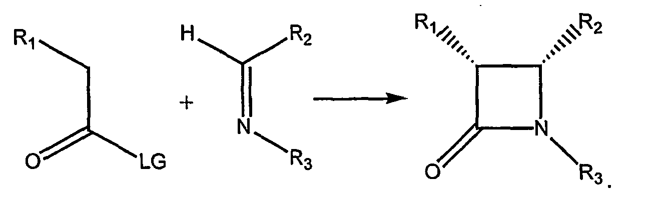 In this scheme, R₁ is hydroxyl, protected hydroxyl, thiol, or protected thiol; LG is a leaving group; R₂ is alkyl, alkenyl, alkynyl, or aryl where R₂ is optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and R₃ is hydrogen. In a preferred embodiment, R₁ is thioaryl or substituted thioaryl, *e.g*., thiophenyl or substituted thiophenyl. In one embodiment of the invention, R₁ is thiophenyl. In a preferred embodiment, R₂ is aryl or substituted aryl, *e.g*., phenyl or substituted phenyl. In one embodiment of the invention, R₂ is phenyl. The scheme shows the formation of the cis product (i.e., R₁ and R₂ are cis), however it is typically the case that both the cis and trans products are formed. As one option, the imine may be prepared as shown in Reaction 2, wherein (R₂)(H)C=N-R₃ is prepared by reaction between an aldehyde of the formula R₂-CHO, and an amine of the formula R₃-NH₂.

Reaction 3 shows a specific example of converting an imine to a β-lactam, where this specific conversion is another aspect of the present invention. 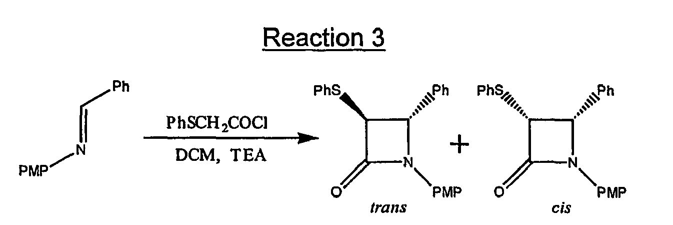 More specifically, an imine is dissolved in an inert solvent such as dichloromethane and cooled to about 0°C under an inert atmosphere such as argon gas. Thiophenyl acetyl chloride or any other respective acid chloride is added dropwise to the cooled stirred solution of the imine at about 0°C. To the resulting solution is added dropwise a tertiary amine, *e.g*., triethylamine, also at about 0°C. The reaction mixture is gradually warmed to room temperature and kept at this temperature for about 16 hours. The reaction is quenched by pouring into ice-cold water and extracted three times with dichloromethane and dried over anhydrous magnesium sulfate. The solvent is evaporated to give the crude product which is purified by column chromatography using dichloromethane initially followed by mixtures of hexane/ethyl acetate to get the pure cis and trans β-lactams shown in Reaction 3. The cis and trans isomers may be separated from one another by, *e.g*., column chromatography. Either isomer, or the mixture of isomers, may be converted to a phenylisoserine compound as described later herein.

Thus, in another aspect, the present invention also provides compounds of the formula 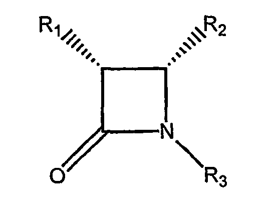 wherein R₁ is thiol (SH), tBOC, acetate, methoxy, thiophenyl, Cl₂CH-C(O)O- or 1-ethoxyethyl, R₂ is phenyl and R₃ is hydrogen.

In another aspect of the invention, an imine without a protecting group attached to the imine nitrogen may be converted to a β-lactam as shown in Reaction 4, where this conversion is another aspect of the invention, and the chemical product is another aspect of the invention. 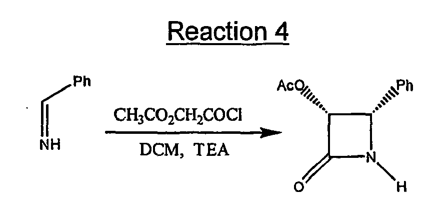 More specifically, to a stirred solution of an imine in an inert solvent such as anhydrous dichloromethane, and preferably under an inert atmosphere such as argon gas, is added acetoxy acetyl chloride dropwise at about 0°C. To this solution is added dropwise a tertiary amine, such as triethylamine, also at about 0°C. The reaction mixture is gradually warmed to room temperature and kept at this temperature for overnight. The reaction is quenched by pouring into ice-cold water and extracted three times with dichloromethane following by drying over anhydrous magnesium sulfate. The solvent is evaporated to give the crude product which may be purified by column chromatography using dichloromethane initially followed by mixtures of hexane/ethyl acetate to give the β-lactam.

### 3. Conversion of an oxime compound to a different oxime compound

In another aspect of the invention, an oxime compound is converted to a protected form as illustrated in Reaction 5.

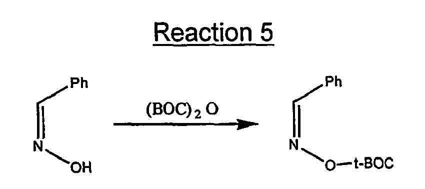

More specifically, a syn-benzaldehyde oxime is added to a stirred solution of NaH in anhydrous THF at 0°C under an argon atmosphere. The reaction mixture is stirred at this temperature for 20 minutes and then (BOC)₂ is added dropwise. The reaction is stirred at 0°C for 1 hr and worked up as usual. The crude product is purified by column chromatography using hexane/dichloromethane to afford the pure product.

### 4. Conversion of an oxime compound to a beta-lactam compound

In another aspect the present invention provides a process for preparing a beta lactam, comprising the scheme 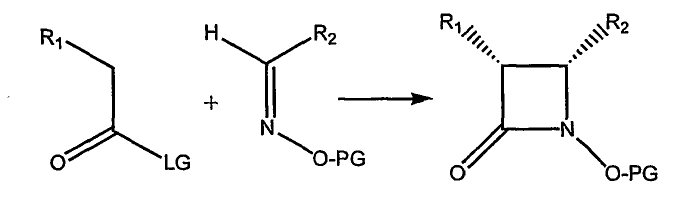 wherein R₁ is hydroxyl, protected hydroxyl, thiol, or protected thiol; LG is a leaving group; R₂ is alkyl, alkenyl, alkynyl or aryl, where R₂ may be optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and PG is a protecting group. Noteworthy is that this process provides beta-lactam compounds having -O-PG substitution at the heterocyclic nitrogen ring.

As an example, in one aspect of the invention, an oxime compound is converted to a beta-lactam.having oxygen substitution on the ring nitrogen, as shown in Reaction 6. 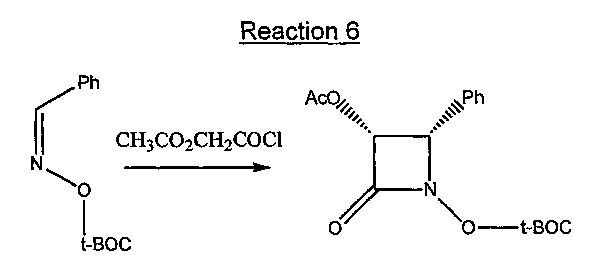 More specifically, a protected oxime is dissolved in dichloromethane and cooled to 0°C under argon atmosphere. Acetoxy acetyl chloride or any other acid chloride is added dropwise to the cooled stirred solution of the oxime at 0°C. To this solution is added dropwise DMAP or any other base also at 0°C. The reaction mixture is gradually warmed to room temperature (or may be heated to about 40°C) and keep at this temperature for 16 hours. The reaction is quenched by pouring into ice-cold water and extracted three times with dichloromethane and dried over anhydrous magnesium sulfate. The solvent is evaporated to give the crude product which is purified by column chromatography using dichloromethane initially followed by mixtures of hexane/ethyl acetate to get the pure product.

Thus, in a related aspect, the present invention provides compounds of the formula 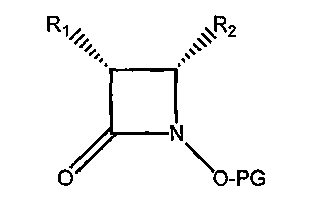 wherein R₁ is hydroxyl, protected hydroxyl, thiol, or protected thiol; R₂ is alkyl, alkenyl, alkynyl or aryl, where R₂ may be optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and PG is a protecting group. Optionally, R₁ is a protected hydroxyl group and the protecting group is selected from methoxymethyl, methoxyethyl, 1-ethoxyethyl, benzyloxymethyl, (beta-trimethylsilyl-ethoxy)methyl, tetrahydropyranyl, 2,2,2-trichloro-ethoxycarbonyl, benzyloxycarbonyl, *tert-*butoxycarbonyl, 9-fluorenylmethoxycarbonyl, 2,2,2-trichloroethoxymethyl, trimethylsilyl, triethylsilyl, tripropylsilyl, dimethylethylsilyl, dimethyl(t-butyl)silyl, diethylmethylsilyl, dimethylphenylsilyl, diphenylmethylsilyl, acetyl, chloroacetyl, dichloroacetyl, trichloroacetyl and trifluoroacetyl. Optionally, R₁ is a protected thiol group, and the protecting group is selected from triphenylmethyl (trityl, Trt), acetamidomethyl (Acm), benzamidomethyl, 1-ethoxyethyl and benzoyl.

### 5. Conversion of a beta-lactam compound to a different beta-lactam compound

A thiophenyl-substituted β-lactam having a protecting group on the ring nitrogen may be deprotected as shown in Reaction 7, where this deprotection reaction is another aspect of the present invention. 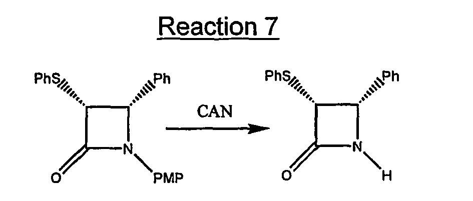 More specifically, cis beta lactam is dissolved in a suitable solvent such as acetonitrile under an inert atmosphere such as argon gas, and cooled to about 0°C. To this stirred cooled solution is added an aqueous solution of ceric ammonium nitrate (CAN) dropwise and the mixture is stirred for about 1 hour. The reaction mixture is poured into water and extracted three times with ethyl acetate. The combined organic phases are successively washed with (a) 5% sodium bicarbonate solution, (b) saturated sodium sulfate solution, and (c) saturated sodium chloride solution, followed by drying over anhydrous sodium sulfite. After evaporation of the solvent under reduced pressure the crude product is purified by column chromatography twice using mixtures of hexane/ethyl acetate and dichloromethane/ethyl acetate to get the pure cis product. The same procedure could also be used to remove the paramethoxy group from trans β-lactam to give the corresponding 3-thiophenyl-azetidinone.

In another aspect, the present invention provides a process whereby the nitrogen atom of a beta-lactam is bonded to a protecting group. This aspect of the invention comprises treating a beta lactam of the structure 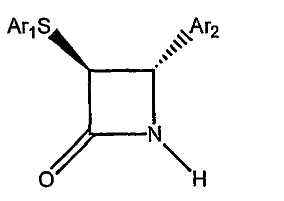 with a base and a protecting agent, to provide a beta lactam of the structure 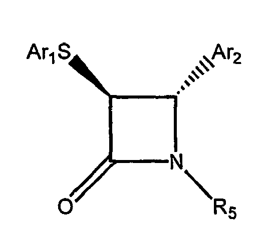 wherein Ar₁ and Ar₂ are aryl groups independently selected at each occurrence, and R₅ is selected from benzoyl and tBOC. The protecting agent may be, for example, benzoyl chloride or di-tert-butyl-dicarbonate. Optionally, this process is proceeded by forming a beta lactam of the formula 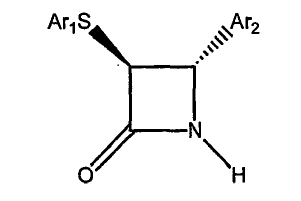 by a process comprising reacting together compounds of the formula Ar₁S-CH₂-C(=O)Cl, base, and Ar₂-CHO under conditions that form the beta lactam. The base may be a nitrogen-containing base, *e.g*., ammonia.

For example, the ring nitrogen of a β-lactam may be protected with an amine protecting group such as benzoyl (Bz, as shown in the following reaction) or t-BOC. This is illustrated in Reaction 8. 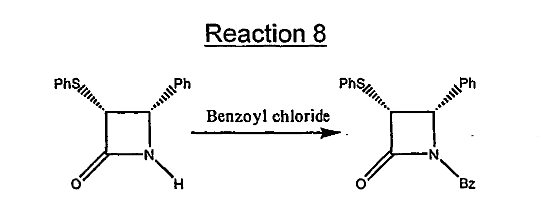 More specifically, a β-lactam is dissolved in an inert solvent such as dichloromethane and cooled to ca. 0°C under an inert atmosphere, *e.g*., argon gas. Dimethylaminopyridine (DMAP) and triethylamine are added followed by dropwise addition of benzoyl chloride at 0°C with stirring. The reaction mixture is stirred for about 1 hour and then was washed with saturated aqueous ammonium chloride and brine and dried over anhydrous sodium sulfate. After removal of the solvent under reduced pressure the crude product is purified by column chromatography using mixtures of dichloromethane/hexane to afford the pure benzoylated β-lactam.

In another aspect of the invention, a paramethoxyphenyl protecting group attached to the ring nitrogen of a β-lactam is replaced with a benzoyl group as shown in Reaction 9. 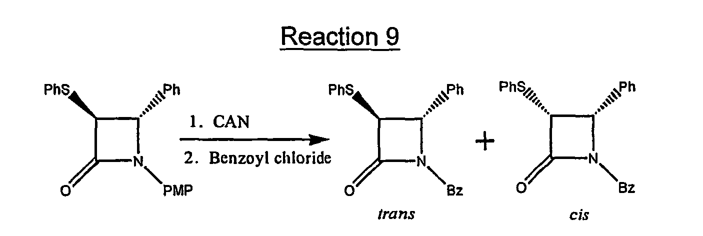 More specifically, the paramethoxy group of the trans β-lactam is removed by using ceric ammonium nitrate (CAN) in aqueous acetonitrile solution, followed by treating the product mixture with benzoyl chloride to afford a mixture of cis and trans benzoylated β-lactam.

In another aspect, the present invention provides for the halogenation of a beta-lactam, as illustrated by the scheme 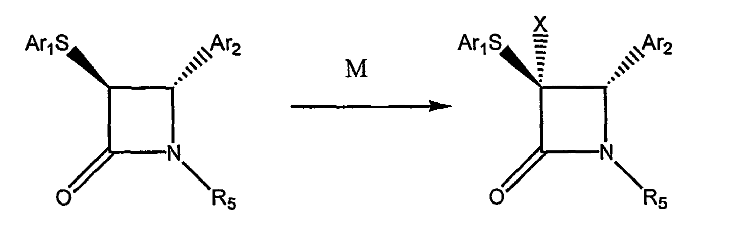 wherein Ar₁ and Ar₂ are each aryl groups, where each of Ar₁ and Ar₂ is independently optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbon atoms, and aryloxycarbonyl where the aryloxy portion contains 6 to 20 carbon atoms; X is halide; R₅ is selected from hydrogen, benzoyl and tBOC, and M is a halogenating agent. In one embodiment, each of Ar₁ and Ar₂ is phenyl. Exemplary halogenating agents include, without limitation, inorganic acid halides, for example thionyl chloride, phosphorus trichloride, phosphorus tribromide, phosphoryl chloride trifluoromethanesulfonic acid, N-iodosuccinimide and phosphorus pentachloride. In one embodiment of the invention, the halogenating agent is SO₂Cl₂.

For example, a trans thiophenyl β-lactam can be modified by introducing a chloro group at the 3-position as shown in Reaction 10. 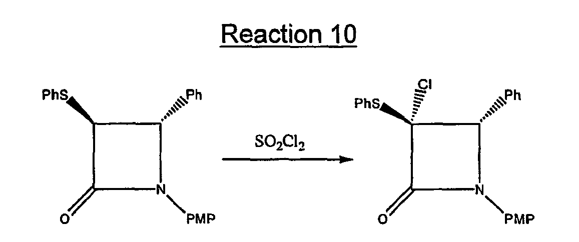 More specifically, a trans thiophenyl beta lactam is dissolved in an inert solvent, *e.g*., anhydrous dichloromethane, under an inert atmosphere, *e.g*., argon gas, and cooled to about 0°C. Sulfuryl chloride is added dropwise to the stirred solution at ca. 0°C and left at this temperature for ca. 2 hrs. The solvent is evaporated and the residue dissolved in dichloromethane and washed successively with water, 10% sodium bicarbonate, saturated brine and dried over anhydrous sodium sulfate. After removal of the solvent under reduced pressure the crude solid is purified by recrystallization using mixtures of dichloromethane/hexanes to give the chloro group at the 3-position of the trans thiophenyl beta lactam.

Thus, the present invention provides compounds of the formula 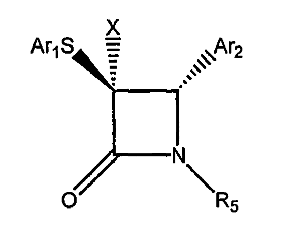 wherein Ar₁ and Ar₂ are each aryl groups, where each of Ar₁ and Ar₂ are independently optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbon atoms, and aryloxycarbonyl where the aryloxy portion contains 6 to 20 carbon atoms; X is halide; and R₅ is selected from hydrogen, benzoyl, tBOC, C₁-C₆ alkyl or aryl where R₅ is optionally substituted with one or more halogens, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms. For example, the invention provides compounds wherein Ar₁ and Ar₂ are each phenyl, X is chloride or bromide; and R₅ is hydrogen, benzoyl or tBOC.

In another aspect, the present invention provides a process wherein a halide substituent on a beta-lactam ring is replaced with a protected hydroxyl group, as illustrated by the following scheme 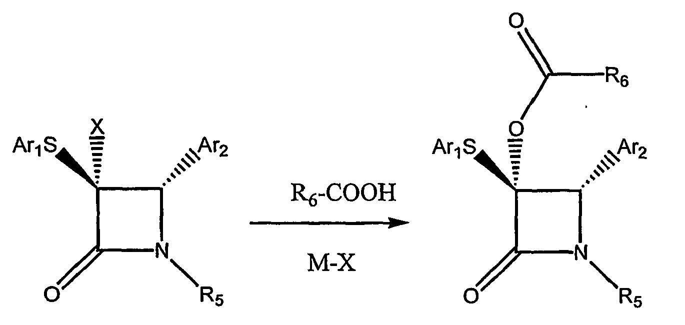 wherein Ar₁ and Ar₂ are each aryl groups, where each of Ar₁ and Ar₂ are independently optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbon atoms, and aryloxycarbonyl where the aryloxy portion contains 6 to 20 carbon atoms; M is metal and X is one or more halides attached to the metal; R₅ is selected from hydrogen, benzoyl and tBOC; and R₆ is C₁-C₆ alkyl. In one exemplary embodiment of this aspect of the invention, Ar₁ and Ar₂ are each phenyl.

For instance, the present invention provides that a chloro-substituted beta-lactam may be converted into the corresponding beta-lactam where the chloride group is replaced with an acetate group. This conversion is illustrated in Reaction 11.

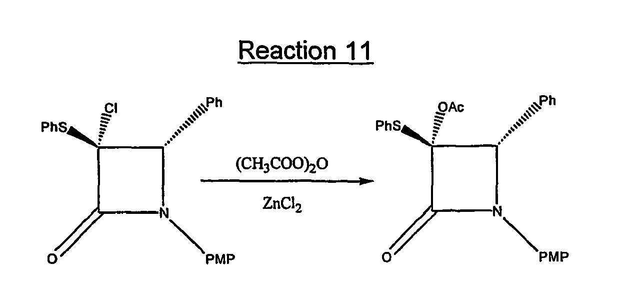

More specifically, the chloro-substituted beta-lactam is dissolved in an inert solvent, *e.g.,* anhydrous dichloromethane, at room temperature under an inert atmosphere, *e.g.,* argon atmosphere. To this stirred solution at room temperature is added sequentially silica gel, zinc chloride and an alkyl anhydride, *e.g.,* acetic anhydride as shown in reaction Xllb. The reaction mixture is left at this temperature for ca. 16 hrs and then worked up. The silica gel is filtered and the filtrate evaporated, dissolved in dichloromethane and worked up as usual for this type of reaction. The crude residue is purified by column chromatography using mixtures of hexanes/ethyl acetate to afford the pure product.

Thus, the present invention provides compounds of the formula 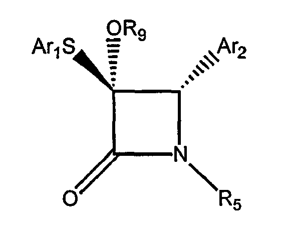 wherein Ar₁ and Ar₂ are each aryl groups, where each of Ar₁ and Ar₂ are independently optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbon atoms, and aryloxycarbonyl where the aryloxy portion contains 6 to 20 carbon atoms; R₅ is selected from hydrogen, benzoyl and tBOC; and R₉ is a hydroxyl protecting group. For instance, in one aspect R₉ is selected from methoxymethyl, methoxyethyl, 1-ethoxyethyl, benzyloxymethyl, (beta-trimethylsilyl-ethoxy)methyl, tetrahydropyranyl, 2,2,2-trichloro-ethoxycarbonyl, benzyloxycarbonyl, *tert-*butoxycarbonyl, 9-fluorenylmethoxycarbonyl, 2,2,2-trichloroethoxymethyl, trimethylsilyl, triethylsilyl, tripropylsilyl, dimethylethylsilyl, dimethyl(t-butyl)silyl, diethylmethylsilyl, dimethylphenylsilyl, diphenylmethylsilyl, acetyl, chloroacetyl, dichloroacetyl, trichloroacetyl and trifluoroacetyl. Alternatively, or in addition, in another aspect Ar₁ and Ar₂ are each phenyl.

In another aspect of the invention, the protecting group of an N-protected beta lactam is replaced with a different protecting group, as shown in Reaction 12. 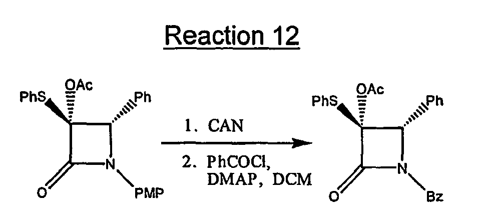 More specifically, a paramethoxyphenyl (PMP) group is cleaved by using the procedure as in Reaction 7. The product obtained from this cleavage is dissolved in an inert solvent, *e.g.*, anhydrous dichloromethane, at ca. room temperature under argon atmosphere. To this stirred solution is added DMAP and dropwise benzoyl chloride, and the reaction is maintained at this temperature for about 1.5 hrs. The reaction mixture is worked up as usual and purified by column chromatography using mixtures of hexanes/ethyl acetate to afford the pure benzoylated beta lactam

In another aspect of the invention, the thiophenyl group of a thiophenyl-substituted beta lactam is removed using a desulfurization reagent, and a hydrogen put in its place. An example is shown in Reaction 13, where the desulfurization reagent is Raney Ni.

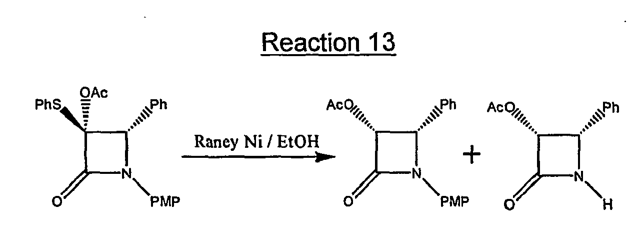

In one specific example, a thiophenyl-substituted beta lactam is dissolved in ethanol at room temperature and Raney nickel is added in one portion to the stirred solution and the reaction mixture is stirred at this temperature for about 2 hrs. The reaction mixture is filtered and the filtrate is evaporated. The residue is dissolved in an inert solvent such as dichloromethane and worked up as usual. The crude product is purified by column chromatography using mixtures of hexanes/ethyl acetate to afford the pure product. Often, the product will be obtained as a mixture of N-protected and N-deprotected beta lactams.

In another aspect of the invention, and as illustrated in Reaction 14, a beta lactam with oxygen substitution on the ring nitrogen is converted to the corresponding beta-lactam with hydrogen substitution on the ring nitrogen.

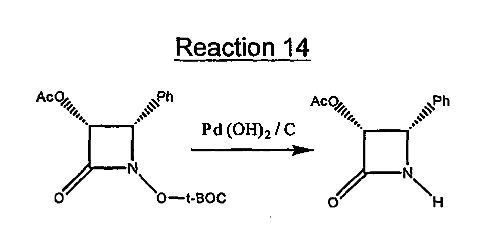

More specifically, a beta lactam with oxygen substitution on the ring nitrogen is dissolved in methanol at room temperature and treated with Pd(OH)₂-C (or any other reducing agent) and the resulting suspension is stirred under hydrogen atmosphere for overnight. The reaction mixture is filtered through a pad of celite and the volatile component(s) of the filtrate are evaporated. The residue was dissolved in dichloromethane and worked up as usual. The crude product is purified by column chromatography using mixtures of hexanes/ethyl acetate to afford the pure beta lactam.

In another aspect, the present invention provides a process comprising the process disclosed in Reaction 15, wherein a thioaryl group is converted to a protected hydroxyl group 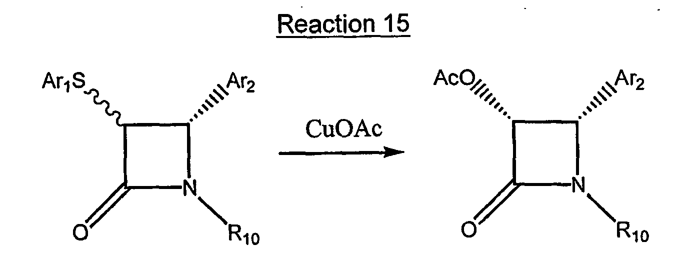 wherein Ar₁ and Ar₂ are independently selected from alkyl, alkenyl, alkynyl, aryl or substituted aryl radical; and R₁₀ is hydrogen, C₁-C₆alkyl, aryl or substituted aryl radical; wherein a substituted aryl radical is substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms.

More specifically, a beta lactam with a phenylthio substitution on the ring is dissolved in an organic solvent at room temperature and treated with copper acetate. The reaction mixture is filtered through a pad of celite and the volatile component(s) of the filtrate are evaporated. The crude product is purified by column chromatography using mixtures of hexanes/ethyl acetate to afford the pure beta lactam.

In another aspect, the present invention provides a process comprising the process disclosed in Reaction 16 wherein a thioaryl group is converted to a hydroxyl group 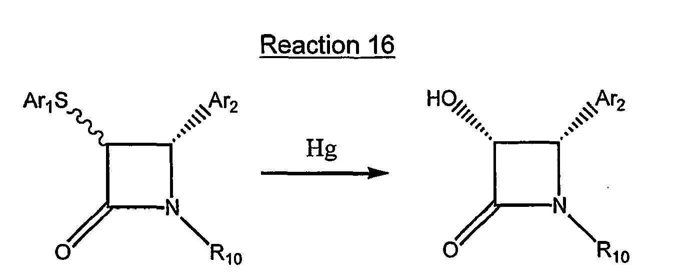 wherein Hg represents a mercuric reagent, *e.g.,* mercuric oxide or mercuric trifluoroacetate, and Ar₁ and Ar₂ are independently selected from alkyl, alkenyl, alkynyl, aryl or substituted aryl radical; and R₁₀ is hydrogen, C₁-C₆alkyl, aryl or substituted aryl radical; wherein a substituted aryl radical is substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms. Optionaly, the mercuric reagent may be combined with ceric ammonium nitrate (CAN).

### 6. Conversion of a beta-lactam compound to a 3-phenylisoserine compound

In another aspect, the present invention provides a process of opening a beta-lactam ring. The process may be illustrated by the following scheme 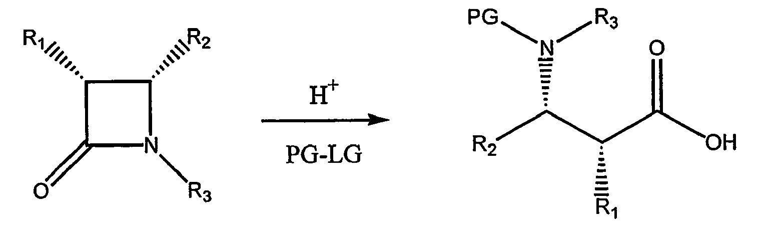 wherein R₁ is hydroxyl, protected hydroxyl, thiol, or protected thiol; LG is a leaving group; PG is an amino protecting group; R₂ is alkyl, alkenyl, alkynyl, or aryl where R₂ is optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; R₃ is hydrogen, C₁-C₆ alkyl or aryl where R₃ is optionally substituted with one or more halogens, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and H⁺ is a proton source. Optionally, the ring-opened product is purified by column chromatography followed by recrystallization, where the recystallization is preferably performed with an organic solvent. The process may be performed in a mixture of organic solvent and aqueous acid. In a preferred embodiment, R₁ is thiophenyl, R² is phenyl, and R³ is hydrogen.

For example, in one embodiment the present invention provides for the conversion of a β3-lactam with thiophenyl substitution to the corresponding phenylisoserine compound as shown in Reaction 17. 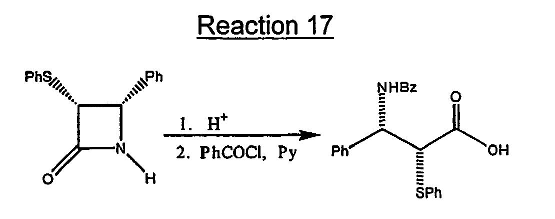 More specifically, a β-lactam is dissolved in a minimum volume of DMSO or mixtures of DMSO/DCM and hydrochloric acid is added. The stirred reaction mixture is heated to about 85°C for ca. 16 hrs. The reaction mixture is cooled to room temperature and dried under vacuum to give a powder, which is the salt of an intermediate compound of the structure 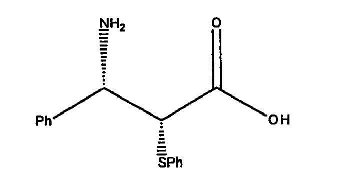 This powder is dissolved in pyridine under an inert atmosphere (*e.g.*, argon) and benzoyl chloride is added dropwise at room temperature. The reaction mixture is stirred at this temperature for about 2 hrs. The reaction mixture is acidified with 0.1N HCl and the crude product is extracted with dichloromethane. The combined organic extracts are dried over anhydrous magnesium sulfate and concentrated *in vacuo* to dryness. The crude product is purified by column chromatography using hexane/ethyl acetate and dichloromethane/ methanol to afford the pure cis phenylisoserine side chain.

In another aspect of the invention, ring-opening of a β-lactam provides a phenylisoserine compound as illustrated in Reaction 18. 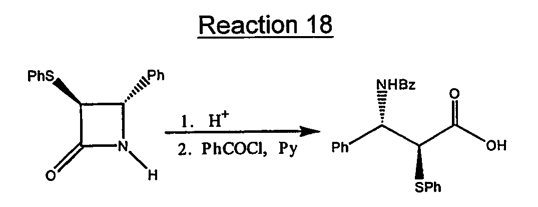 More specifically, treatment of a trans β-lactam with protic acid followed by reaction with benzoyl chloride in base (*e.g.*, pyridine) affords a trans phenylisoserine side chain.

In another aspect, the present invention provides a process wherein a beta-lactam having both thiophenyl and protected hydroxyl substitution is converted to a ring-opened form, as illustrated by the following scheme 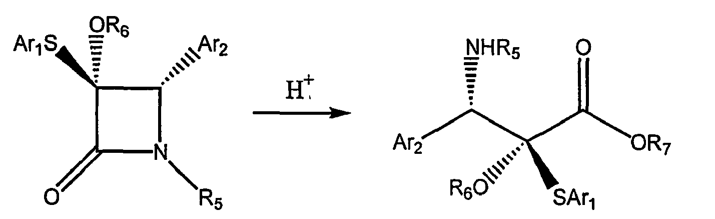 wherein Ar₁ and Ar₂ are aryl groups independently selected at each occurrence, R₅ is selected from hydrogen, benzoyl and tBOC, R₆ is a hydroxy protecting group, and R₇ is hydrogen or C₁-C₆alkyl, where R₇ as C₁-C₆alkyl is introduced in an optional esterification reaction. H+ represents a proton source, *e.g*., mineral acid or organic acid. In one aspect of the invention, Ar₁ and Ar₂ are each phenyl.

In a separate aspect, the present invention provides a process of opening a beta lactam according to the scheme 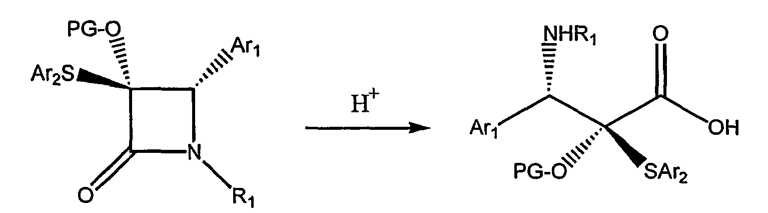 wherein PG is a hydroxyl protecting group; Ar₁ and Ar₂ are each aryl groups, where each of Ar₁ and Ar₂ are independently optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbon atoms, and aryloxycarbonyl where the aryloxy portion contains 6 to 20 carbon atoms; and R₁ is hydrogen, alkyl, or -O-PG wherein PG is a protecting group.

For example, in one aspect of the invention, a beta-lactam is ring-opened to afford the corresponding phenylisoserine compound as shown in Reaction 19. 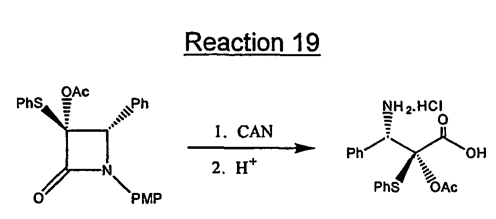 More specifically, the paramethoxyphenyl (PMP) group of the beta-lactam shown in Reaction 19 is cleaved by using the procedure as in Reaction 7. The product obtained from this cleavage is dissolved in a minimum volume of dichloromethane at room temperature and a solution of hydrochloric acid is added. The stirred solution is heated to about 60°C for about 3 hrs. The reaction mixture is cooled to room temperature and concentrated *in vacuo* to dryness, giving the acid as a powder.

In another aspect, the present invention provides a process whereby a beta lactam having oxygen substitution on the ring nitrogen is converted into a phenylisoserine compound, as illustrated in Reaction 20. 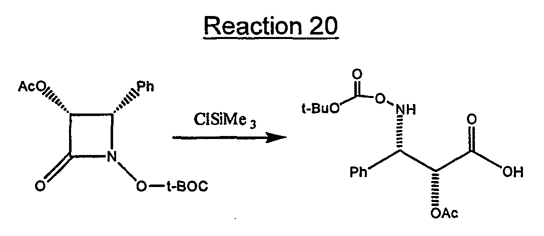 More specifically, a beta lactam having oxygen substitution on the ring nitrogen is dissolved in dichloromethane at room temperature under argon atmosphere and TMSCI is added. This solution is stirred for about 4 hrs and worked up as usual. The combined organic extracts are dried over anhydrous magnesium sulfate and concentrated *in vacuo* to dryness to give a solid product.

Thus, the present invention generally provides isoserine compound of the formula 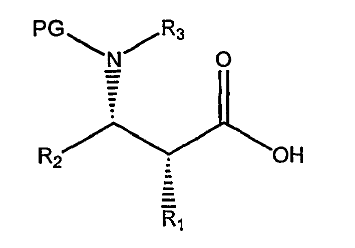 wherein R₁ is hydroxyl, protected hydroxyl, thiol, or protected thiol; PG is an amino protecting group; R₂ is alkyl, alkenyl, alkynyl, or aryl where R₂ is optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; R₃ is hydrogen, C₁-C₆ alkyl or aryl where R₃ is optionally substituted with one or more halogens, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and salts and esters thereof. In one aspect, the isoserine compound is characterized by having R₁ be hydroxyl or protected hydroxyl; R₂ be aryl; and R₃ be hydrogen; including salts and esters thereof. In another aspect, the isoserine compound is characterized by having R₁ be thiol or protected thiol; R₂ be aryl; R₃ be hydrogen; and includes salts thereof.

In addition, the present invention provides compounds of the formula 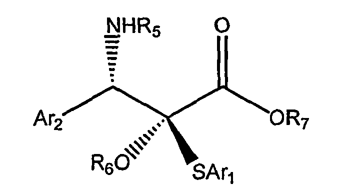 wherein Ar₁ and Ar₂ are aryl groups independently selected at each occurrence, R₅ is selected from hydrogen, benzoyl and tBOC, R₆ is a hydroxyl protecting group, and R₇ is hydrogen or C₁-C₆alkyl. Optionally, R₆ is selected from methoxymethyl, methoxyethyl, 1-ethoxyethyl, benzyloxymethyl, (beta-trimethylsilyl-ethoxy)methyl, tetrahydropyranyl, 2,2,2-trichloro-ethoxycarbonyl, benzyloxycarbonyl, *tert-*butoxycarbonyl, 9-fluorenylmethoxycarbonyl, 2,2,2-trichloroethoxymethyl, trimethylsilyl, triethylsilyl, tripropylsilyl, dimethylethylsilyl, dimethyl(t-butyl)silyl, diethylmethylsilyl, dimethylphenylsilyl, diphenylmethylsilyl, acetyl, chloroacetyl, dichloroacetyl, trichloroacetyl and trifluoroacetyl.

Furthermore, the present invention provides isoserine compounds of the formula 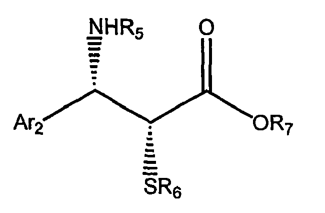 wherein Ar₂ is an aryl group R₅ is selected from hydrogen, benzoyl and tBOC, R₆ is a thiol protecting group, and R₇ is H or C₁-C₆ alkyl. Optionally, the thiol protecting group is triphenylmethyl (trityl, Trt), acetamidomethyl (Acm), benzamidomethyl, 1-ethoxyethyl or benzoyl.

### 7. Conversion of a 3-phenylisoserine compound to another 3-phenylisoserine compound

In one aspect, the present invention provides a process whereby a thioaryl group in a phenylisoserine compound is replaced with a hydroxyl group, as shown in the following Reaction 21. 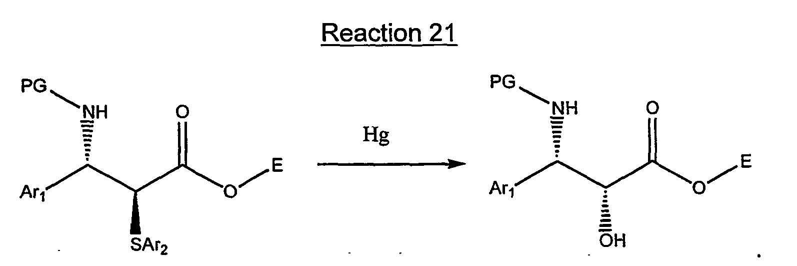 In Reaction 21, PG is an amine protecting group, Ar₁ and Ar₂ are aryl groups, E is hydrogen or an organic group, and Hg represents a mercury-containing oxidizing agent. Optionally, PG is benzoyl or tBOC, and/or E is hydrogen, and/or Ar₁ is phenyl, and/or Ar₂ is phenyl. Two exemplary mercuric oxidizing agents are HgO and Hg(CF₃CO₂)₂.

For example, the present invention provides that a thiophenyl group located at the 2-position of a 3-phenylisoserine may be replaced with a hydroxyl group of the opposite configuration, as shown in Reaction 22. 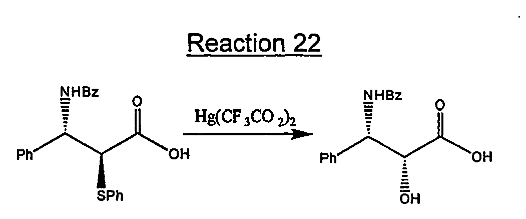 More specifically, a trans 2-thiophenyl 3-phenylisoserine compound is dissolved in an inert solvent, *e.g.*, freshly distilled THF, under an inert atmosphere, *e.g.,* argon gas, and a mercury-containing oxidizing agent, *e.g.,* mercuric oxide (HgO) or Hg(CF₃CO₂)₂ as shown in Reaction 22, is added in one portion at room temperature and the reaction mixture stirred at this temperature for about 72 hrs. The reaction is worked up according to procedures known in the art for reactions with mercuric oxidizing agent, and the product is purified by column chromatography using mixtures of acetone/methanol to afford the pure cis phenylisoserine side chain.

In another aspect, the present invention provides a process whereby a hydroxyl group in a phenylisoserine compound is converted to a protected hydroxyl group, as shown in Reaction 23. 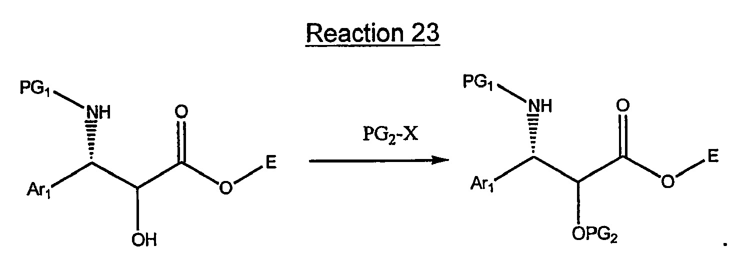 In Reaction 23, PG₁ is an amine protecting group, Ar₁ and Ar₂ are aryl groups, E is hydrogen or an organic group, PG₂ is a hydroxyl protecting group, and PG₂-X represents a reagent that introduces a protecting group onto a hydroxyl group. Optionally, PG₁ is benzoyl or tBOC, and/or E is hydrogen, and/or Ar₁ is phenyl, and/or Ar₂ is phenyl and/or PG₂ is acetyl. An exemplary reagent to add a protecting group onto a hydroxyl group is acetyl chloride. Other reagents are well known in the art, including those set forth in T. W. Greene and P. G. M. Wuts, "Protective Groups in Organic Synthesis," Second Edition, John Wiley and Sons, New York, N.Y., 1991, Chapters 2 and 3.

For example, the present invention provides for the acylation of the 2-hydroxy group of a 3-phenyl-2-hydroxy isoserine compound, as shown in Reaction 24. 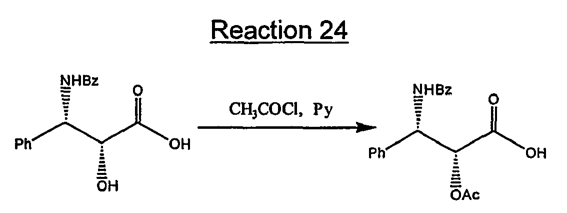 More specifically, a cis phenylisoserine compound is dissolved in a basic solvent, *e.g.,* pyridine, under an inert atmosphere, *e.g.,* argon gas, at about room temperature and acetyl chloride is added dropwise to the stirred solution. The solution is stirred for about 30 minutes and worked up according to methods known in the art for acylation reaction. The crude product is purified by column chromatography using mixtures of dichloromethane/methanol to afford the pure acetylated cis phenylisoserine side chain acid.

In another aspect, the present invention provides a process whereby a thioaryl group is removed from an arylisoserine compound, as illustrated by the scheme 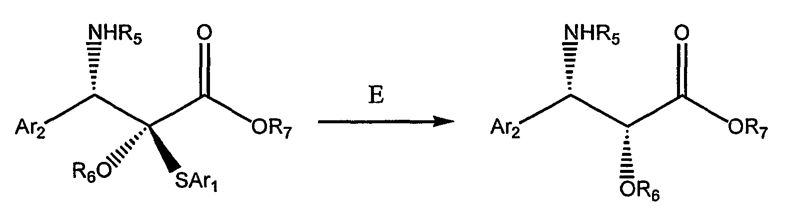 wherein Ar₁ and Ar₂ are aryl groups independently selected at each occurrence, R₅ is selected from hydrogen, benzoyl and tBOC, R₆ is C₁-C₆ alkyl, R₇ is H or C₁-C₆ alkyl, and E represents a desulfuration reagent. Raney nickel is a suitable desulfurization reagent. In a preferred embodiment, each of Ar₁ and Ar₂ is phenyl. For example, the present invention provides a process whereby a thioaryl group is removed from an arylisoserine compound as illustrated by the scheme of Reaction 25. 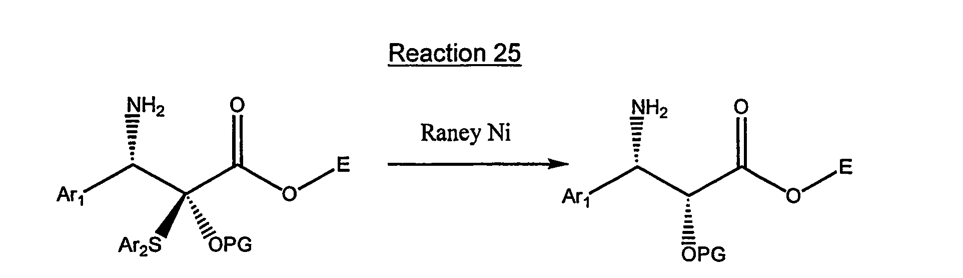 In the above scheme, Ar₁ and Ar₂ are aryl groups, E is hydrogen or an organic group, and OPG represents a protected hydroxyl group. Optionally, Ar₁ is phenyl, and/or Ar₂ is phenyl, and/or E is hydrogen and/or PG is acetyl or ethoxyethyl (EE).

Thus, the present invention provides compounds of the formula 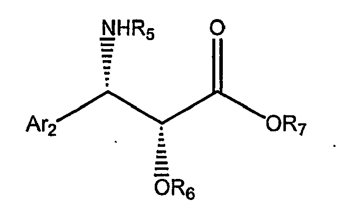 wherein Ar₂ is an aryl group R₅ is selected from hydrogen, benzoyl and tBOC, R₆ is a hydroxyl protecting group, and R₇ is H or C₁-C₆ alkyl. Optionally, R₆ is selected from methoxymethyl, methoxyethyl, 1-ethoxyethyl, benzyloxymethyl, (beta-trimethylsilyl-ethoxy)methyl, tetrahydropyranyl, 2,2,2-trichloroethoxycarbonyl, benzyloxycarbonyl, *tert*-butoxycarbonyl, 9-fluorenylmethoxycarbonyl, 2,2,2-trichloroethoxymethyl, trimethylsilyl, triethylsilyl, tripropylsilyl, dimethylethylsilyl, dimethyl(t-butyl)silyl, diethylmethylsilyl, dimethylphenylsilyl, diphenylmethylsilyl, acetyl, chloroacetyl; dichloroacetyl, trichloroacetyl and trifluoroacetyl.

In another aspect, the present invention provides a process whereby a protecting group is added to the amino group of an arylisoserine compound, as illustrated in the scheme of Reaction 26. 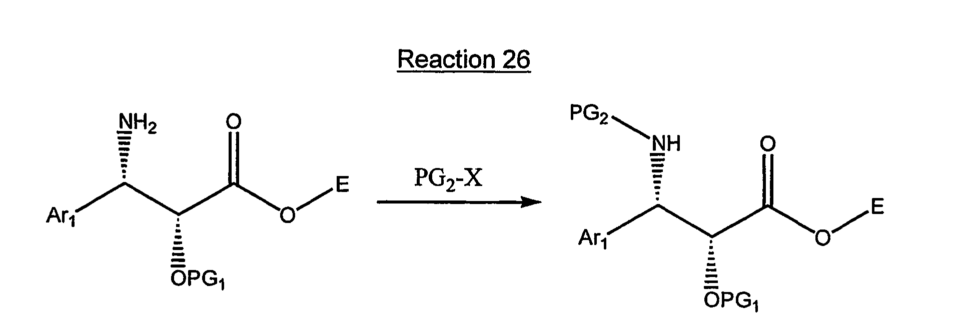 In Reaction 26, Ar₁ and Ar₂ are aryl groups, E is hydrogen or an organic group, PG₁ represents a hydroxyl protecting group and PG2 represents an amine protecting group. Optionally, Ar₁ is phenyl, and/or Ar₂ is phenyl, and/or E is hydrogen and/or PG₁ is acetyl. Optionally, when paclitaxel is the target taxane, PG₂ is a benzoyl group. However, when taxotere is the target taxane, then PG₂ is a tBOC group.

In another aspect of the present invention, a protecting group is added to the amine group of a 3-arylisoserine compound, and a thioaryl group is removed from the alpha carbon, as illustrated in Reaction 27, where phenyl is shown as a representative aryl group, acetate is shown as a representative hydroxyl protecting group, and benzoyl is shown as a representative amine protecting group. 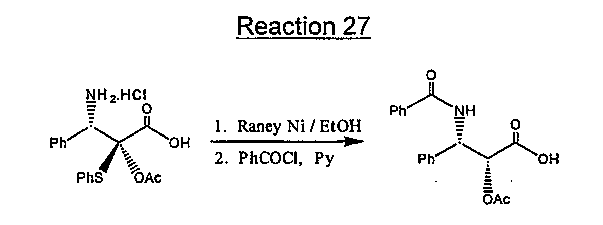 More specifically, a phenylisoserine compound is dissolved in ethanol at room temperature and Raney nickel is added in one portion to the stirred solution and the reaction mixture is stirred at this temperature for 3 hrs. The reaction mixture is filtered and the filtrate is evaporated. The residue is dissolved in dichloromethane and worked up as usual. This resulting solid is dissolved in pyridine under argon atmosphere and benzoyl chloride added dropwise at room temperature. The reaction mixture is stirred at this temperature for about 4 hrs. The reaction mixture is acidified with 0.1N HCl and the crude product is extracted with dichloromethane. The combined organic extracts are dried over anhydrous magnesium sulfate and concentrated *in vacuo* to dryness. The crude product is purified by column chromatography using dichloromethane/methanol to afford the pure cis 2'-acetylated phenylisoserine side chain. When taxotere is the target taxane, a reagent that adds a tBOC group to an amine group may be used in lieu of benzoyl chloride.

In another aspect of the invention, the protecting group on the nitrogen atom of a 3-phenylisoserine compound is replaced with a different protecting group as illustrated in Reaction 28. 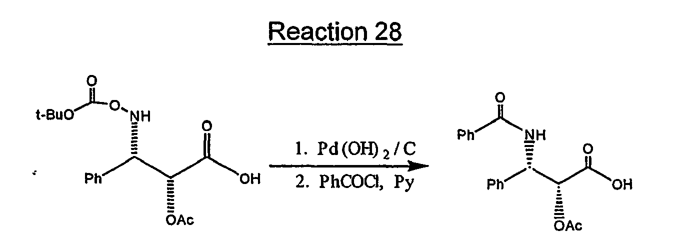 Here, a O-t-BOC protected phenylisoserine compound is treated under reducing conditions as shown in reaction 28, and then benzoylated using benzoyl chloride in pyridine according to reaction 27 to give the 2'-protected phenylisoserine taxol side chain.

### B. Combinations of Reactions

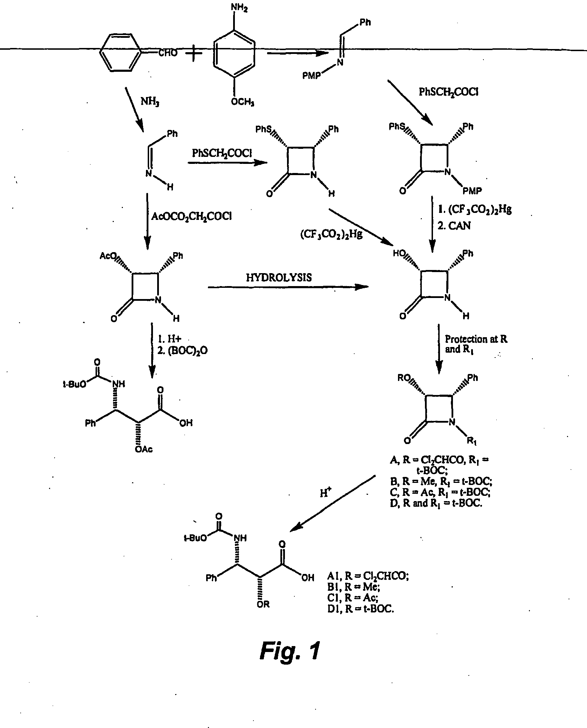

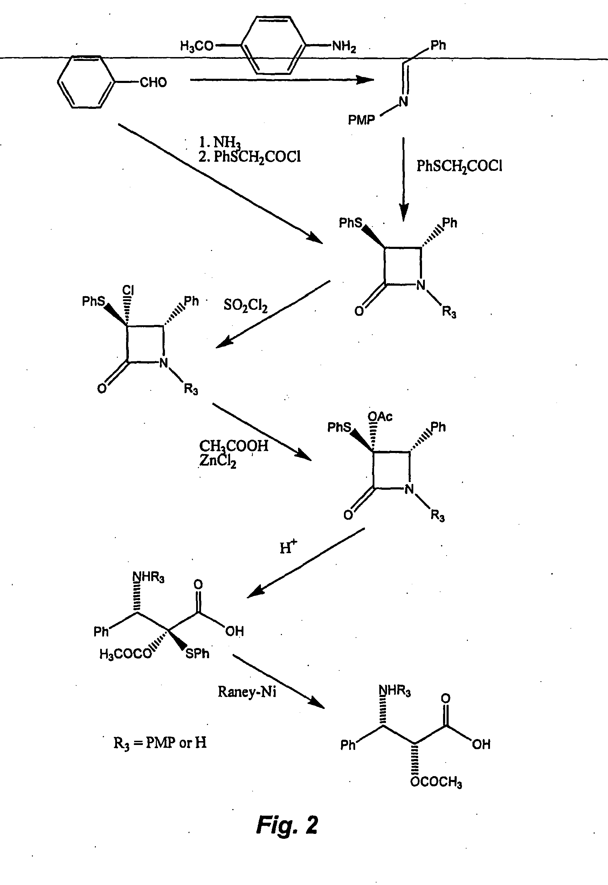

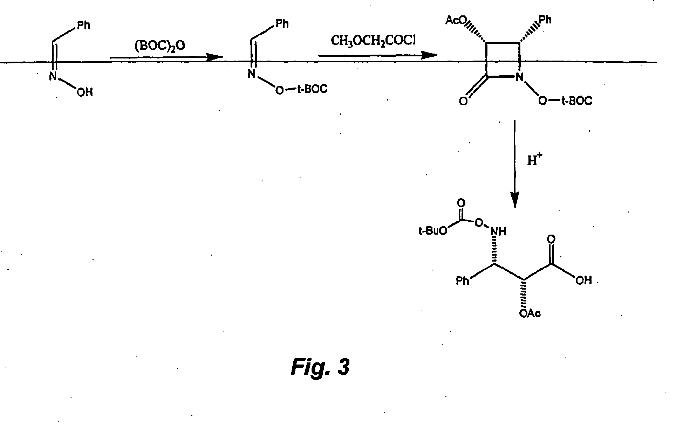

The various reactions described in this section may be carried out sequentially, so long as the product of one reaction may be used as the starting material of another reaction. Each of these possible combinations is a separate aspect of the present invention. Exemplary reaction sequences are shown in Figures 1-3.

### C. Baccatin III Compounds

### C7-dichloroacetyl baccatin III

In one aspect the present invention provides C7-dichloroacetyl baccatin III of the following formula (R₇ = -OCOCHCl₂). 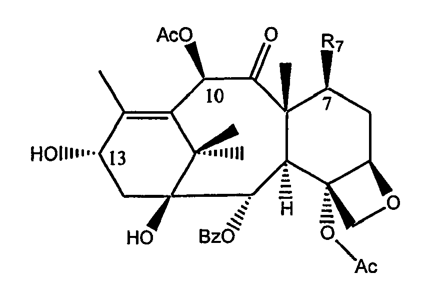 This compound is a useful intermediate in the production of taxanes. This compound may be prepared according to Reaction 29, which is another aspect of the present invention.

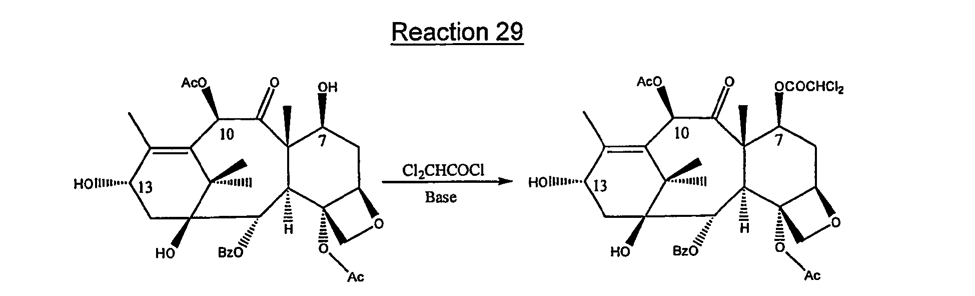

In Reaction 29, the base may be an amine base, e.g., dimethylaminopyridine (DMAP). The reaction is typically conducted in an inert solvent, e.g., dichloromethane (DCM). For example, Baccatin III may be dissolved in anhydrous dichloromethane under an argon atmosphere at room temperature. To this solution is added DMAP followed by dichloroacetyl chloride. The mixture is left at room temperature for overnight. The mixture is then quenched with cold water and extracted thrice with dichloromethane. The organic layer is washed with water and than with brine to remove unwanted salts. The organic layer may then be dried and evaporated under vacuum, and the residue recrystallized or column chromatographed with dichloromethane/ethyl acetate mixtures to afford C7 protected baccatin III.

Alternatively, the C7 protected baccatin III or C7 and C10 protected baccatin III can also be prepared from 10 DAB or 9DHB (9-dihydro-13-acetylbaccatin III) in a similar manner.

### C7-triethylsilyl baccatin III

In one aspect the present invention provides C7-triethylsilyl baccatin III of the following formula (R₇ = -O-Si(CH₂CH₃)₃). 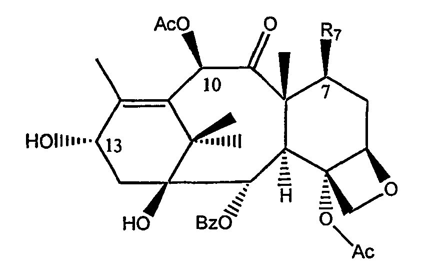 This compound is a useful intermediate in the production of taxanes. This compound may be prepared according to Reaction 30, which is another aspect of the present invention.

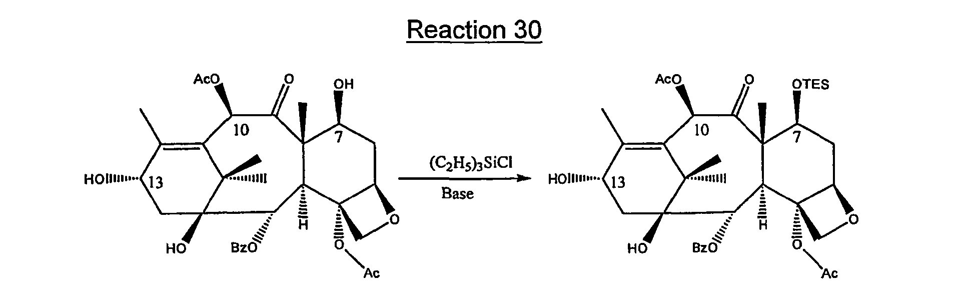

In Reaction 30, the base may be an amine base, *e.g*., dimethylaminopyridine (DMAP) or pyridine. The reaction is typically conducted in an inert solvent, *e.g*., dichloromethane (DCM). For example, Baccatin III may be dissolved in anhydrous dichloromethane under an argon atmosphere at room temperature. To this solution is added pyridine followed by triethylsilyl chloride. The mixture is left at room temperature for overnight. The mixture is then quenched with cold water and extracted thrice with dichloromethane. The organic layer is washed with water and than with brine to remove unwanted salts. The organic layer may then be dried and evaporated under vacuum, and the residue recrystallized or column chromatographed with dichloromethane/ethyl acetate mixtures to afford C7 protected baccatin III.

### A. Condensation of the C7 Protected Baccatin III with the Side Chain

In another aspect, the present invention provides for the coupling of a sidechain as described in the previous section, which may be either a beta lactam or a phenylisoserine, with a baccatin-type compound. In general, the baccatin-type compound is described by the formula 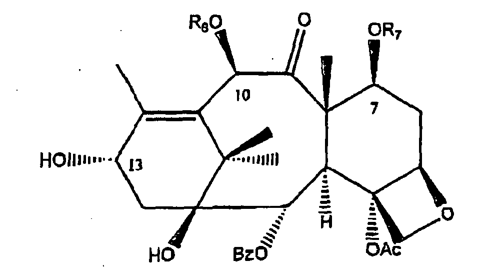 wherein R₆ and R₇ are selected from hydrogen and hydroxy protecting groups. The sidechain couples to the baccatin-type compound at the hydroxyl group located at C13 of the baccatin-type compound. In various exemplary embodiments of the invention: R₆ is acetyl and R₇ Is triethylsily (TES); R₆ is acetyl and R₇ is -COCHCl₂; R₆ is dichloroacetyl and R₇ is triethylsily (TES);or R₈ is dichloroacetyl and R₇ is -COCHCl₂. In a preferred embodiment, the coupling is performed in the presence of a dialkylcarbodiimide, e.g., dicyclohexylcarbodiimide.

In one embodiment, a di-chloroacetyl baccatin III (R₇ = - OCOCHCl₂) or -O-triethylsilyl (-O TES) baccatin III of the following formula (I"): 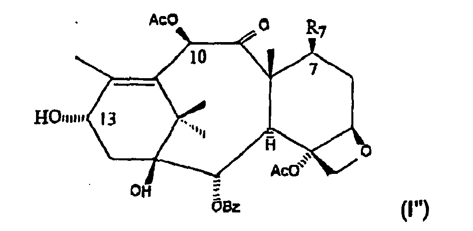 is reacted with an N-CBz C2'-protected 3-phenylisoserine side chain of the following formula (IIa"), or with a β-lactam of the following formula (IIb"): 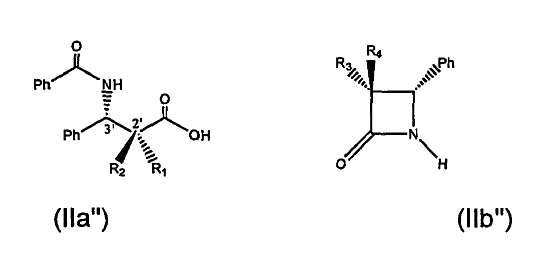 to form an intermediate of the following formulas (III") or (IV"):

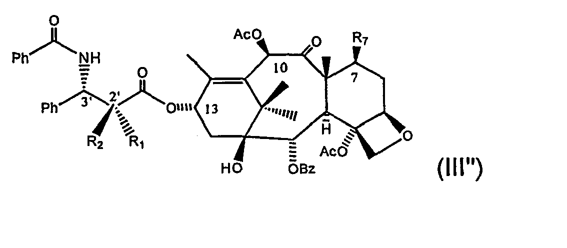

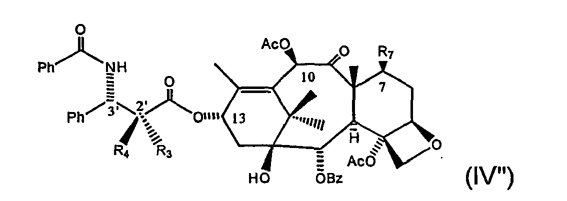

In another embodiment, the intermediate of formula (III") or (IV") is further modified to yield paclitaxel or analogs thereof. For example, the R₇ group at the C7 position and the R group at the C2' site may be converted to hydroxyl groups to yield paclitaxel. In one embodiment of the invention, these coupling reactions are accomplished under the influence of a dialkylcarbodiimide, *e.g*., DCC.

In general, reaction of a beta lactam (see, *e.g*., Reactions 6, 8, 9, 12, and 13) or a phenylisoserine side chain (see, *e.g*., Reactions 10, 11, 18, 20, 22 and 24) may be accomplished by reacting with a C7 protected baccatin III (Schemes I and II below) to yield an intermediate of the following formula (IIIa") or (IIIb") or (IVa") or (IVb"): 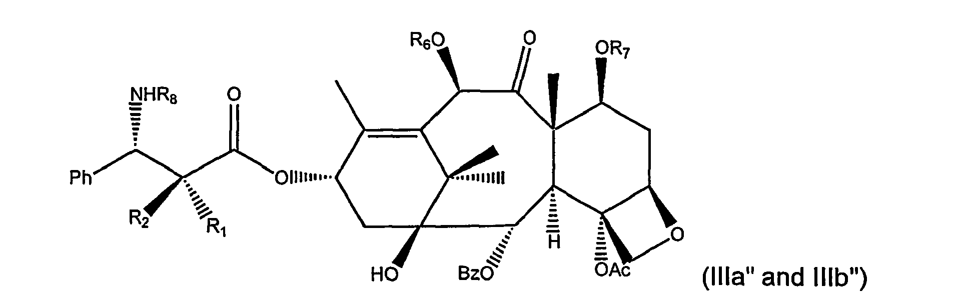 wherein R₆ is acetyl, R₇ is a hydroxy protecting group, and R₈ is benzoyl (compound IIIa") or t-BOC (compound IIIb"); and 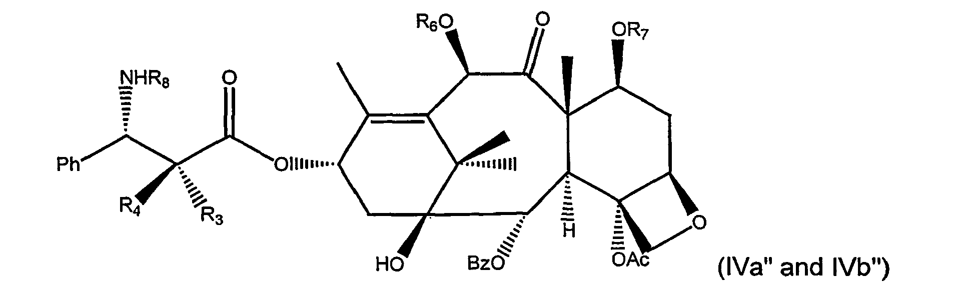 wherein R₆ is acetyl, R₇ is a hydroxy protecting group, and R₈ is benzoyl (compound IVa") or t-BOC (compound IVb").

Such reaction between compounds of formulas (I") and those of formulas (IIa") and (IIb") may be accomplished as illustrated in following reaction Schemes.

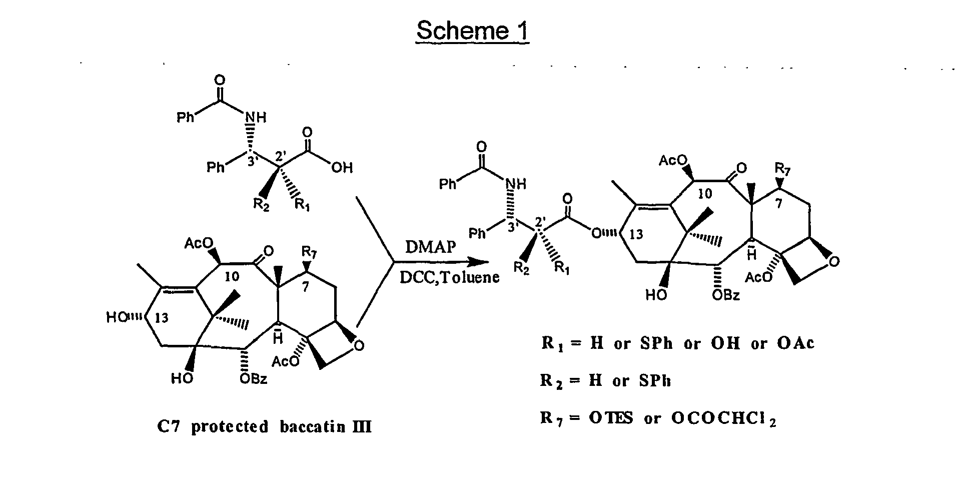

Here, the side chain acid of formula IIa" (obtained as described previously) is dissolved in anhydrous toluene under argon atmosphere at room temperature. To this stirred solution of the side chain acid is added sequentially DCC, DMAP and the C7 protected baccatin III of formula I". The resulting mixture is then heated at about 75°C for 16 hrs. It should be noted that any other dialkycarbodiimides may be substituted for the dicyclohexylcarbodiimide (DCC), with one example being diisopropylcarbodiimide. The solution is then allowed to cool to room temperature, and next an equal volume of dichloromethane is added. The combined organics are then washed with cold dilute hydrochloric acid solution, water, and finally brine. The organic layer is separated, dried, and reduced under vacuum. The resulting residue is purified by column chromatography using mixtures of .dichloromethane/ethyl acetate or hexanes/ethyl acetate to afford the pure coupled intermediate taxane of formula III" or IV".

The process illustrated by Scheme 1 is suited for the preparation of paclitaxel since the sidechain amino group is protected with a benzoyl group. In another embodiment of the invention (not illustrated) the process of Scheme 1 is performed with a sidechain having a t-BOC protecting group for the sidechain amino group, where this embodiment is well-suited for the preparation of taxotere.

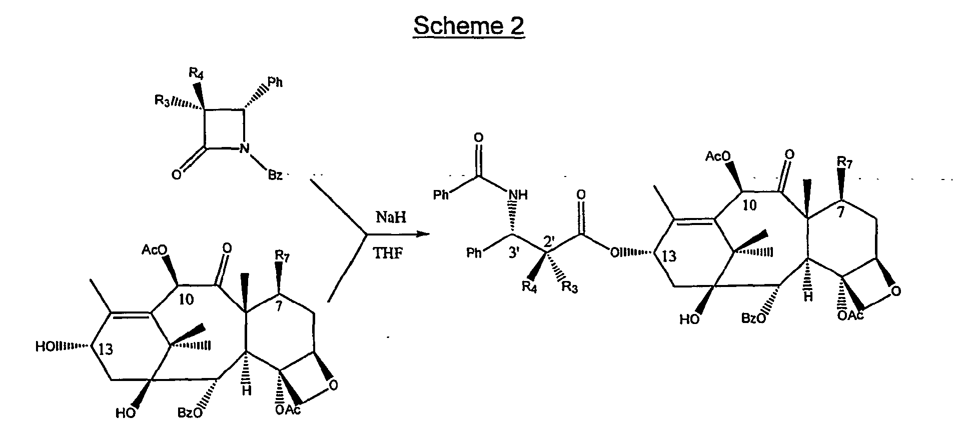

In Scheme 2, in preferred embodiments, R³ is H, SPh, OH, OAc or ethoxyethyl, R⁴ is H or SPh, and R⁷ is O-TES or OCOCHCl₂. Here, the beta lactam of formula IIb" (obtained as described previously) and the C7 protected baccatin III is dissolved in anhydrous freshly distilled THF under argon atmosphere at room temperature. This stirred solution is cooled to 0°C and added to a suspension of NaH in THF at 0°C. The solution is warmed slowly to room temperature and maintained at this temperature for 3 hrs. The reaction mixture was cooled to 0°C and quenched with brine. The reaction mixture was extracted with dichloromethane and the combined extracts were washed several times with brine, dried over anhydrous sodium sulfate, and concentrated under reduced pressure to give the crude product. The crude product was purified by column chromatography using mixtures of hexanes/ethyl acetate to afford the pure coupled intermediate taxane of formula III" or IV" that could be converted to taxol or its analogs. Although this reaction is illustrated with sodium hydride, in other aspects of the invention the coupling is performed in the presence of a metal base salts, *e.g*., a metal hexamethyldisilazide (e.g., LiHMDS, NaHMDS, KHMDS), or a Lewis acid, *e.g*., boron trifluoride etherate.

The process illustrated by Scheme 2 is suited for the preparation of paclitaxel since the nitrogen atom of the beta-lactam is protected with a benzoyl (Bz) group. In another embodiment of the invention (not illustrated) the process of Scheme 2 is performed with the nitrogen atom of the beta-lactam being protected by a t-BOC protecting group, where this embodiment is well-suited for the preparation of taxotere.

Additional examples of the coupling of a sidechain to a baccatin-type compound are shown in the following Schemes 3 and 4. Each of Schemes 3 and 4 is a separate aspect of the present invention. In these schemes "R" represents hydrogen or an organic group, e.g., R may be hydroxyl, protected hydroxyl, thiol, or protected thiol; alternatively R may be alkyl, alkenyl, alkynyl or aryl, where R may be optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms. Preferably, "R" is selected as appropriate for the preparation of paclitaxel or taxotere. DCC is shown as the coupling reagent in Schemes 3 and 4 for illustrative purpoes, however, other dialkylcarbodiimides may be used in lieu of, or in combination with, dicyclohexylcarbodiimide (DCC).

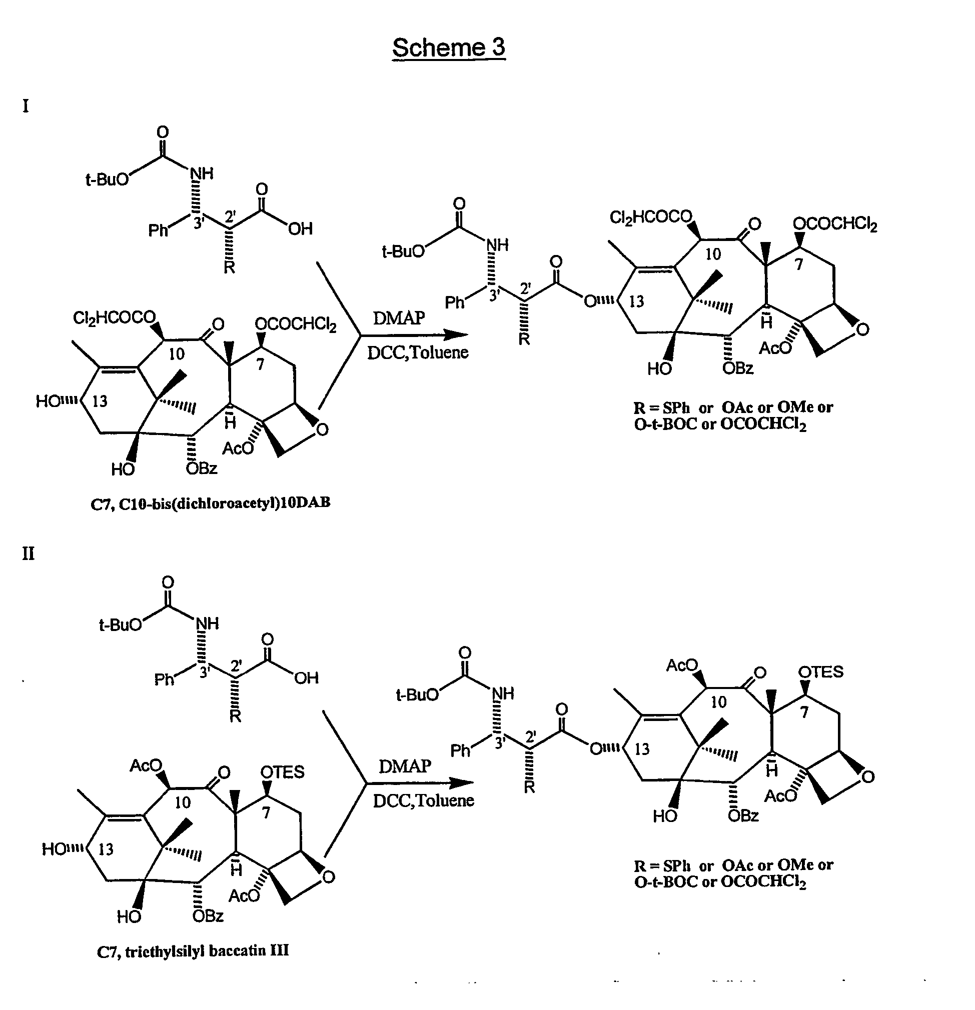

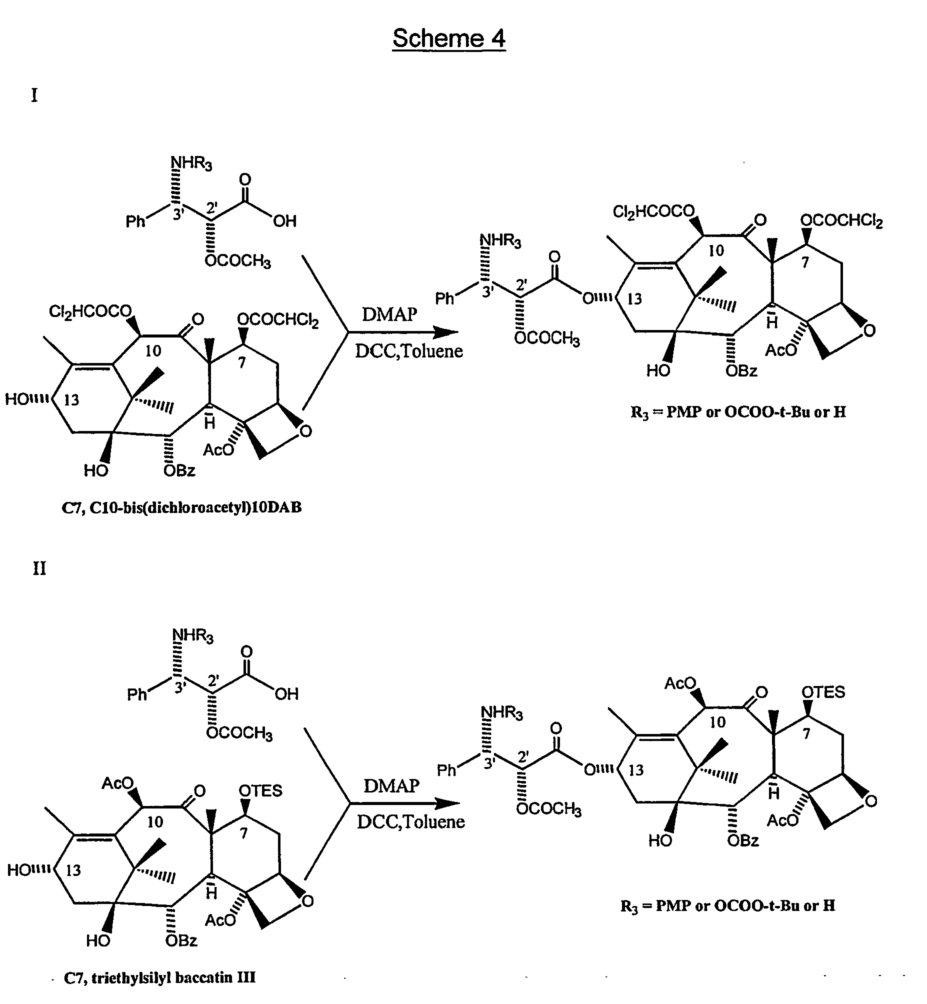

### E. Conversion of the compound of formula III or IV to paclitaxel, taxotere, or an analog thereof

Following synthesis of compounds of formula III" or IV", the same may then be used as an intermediate for the preparation of paclitaxel, taxotere, or analogs thereof. For example, the following Scheme 5 illustrates hydrolysis of the C2'-protected groups and C7-dichloroacetyl or TES to form paclitaxel under mild conditions, thus not disturbing the ester linkage and various substituents.

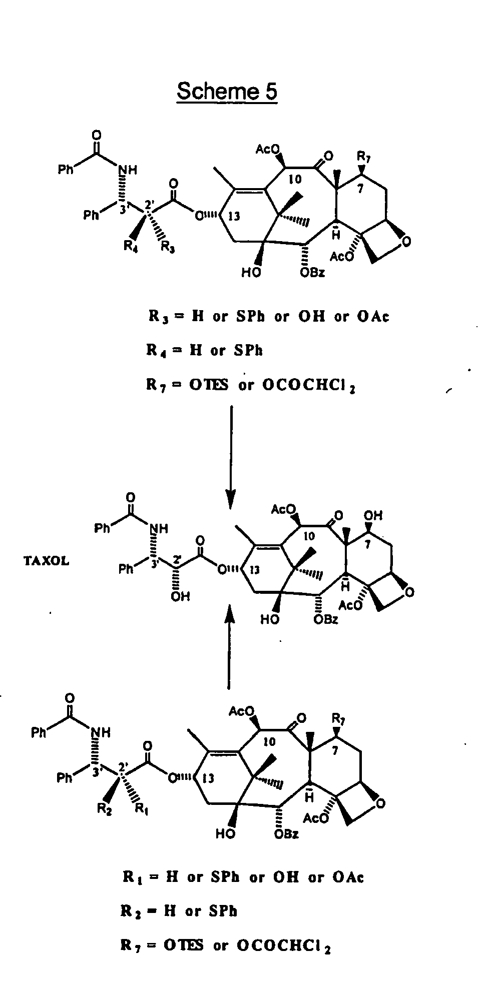

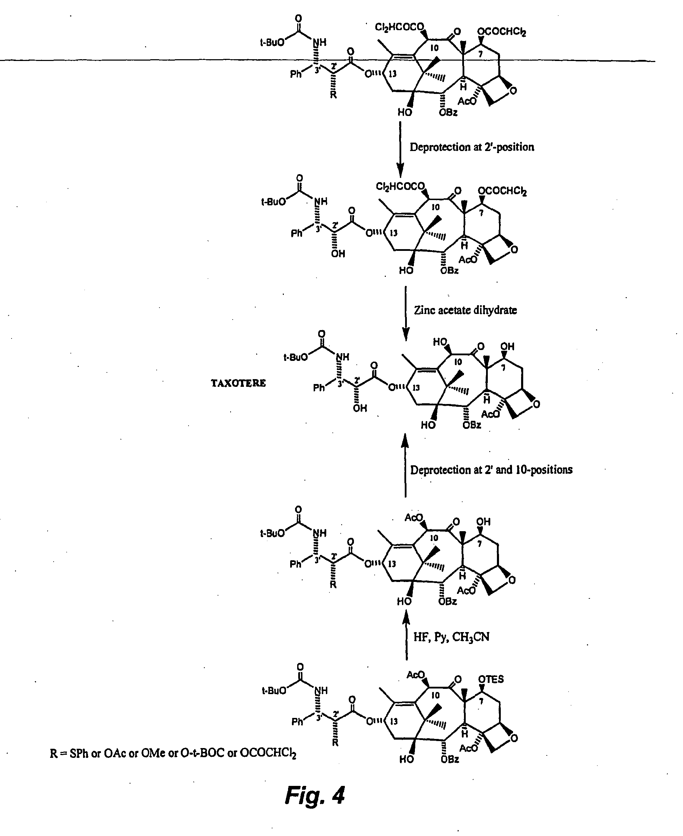

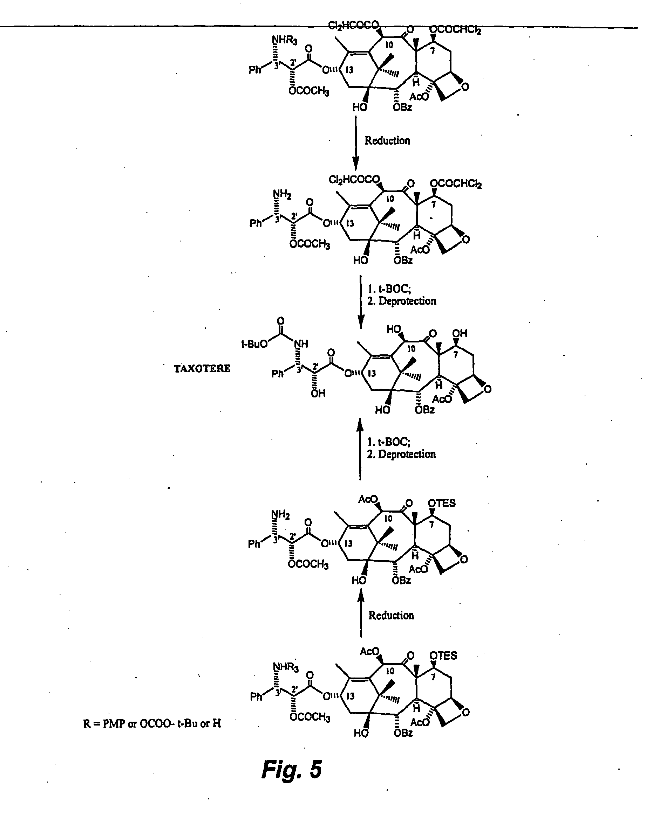

Here, the C2' protected groups and the C7 protected groups can be removed to give taxol or its analogs. An analogous process of the present invention for the preparation of taxotere is shown in Figures 4 and 5.

From the foregoing it will be appreciated that, although specific embodiments of the invention have been described herein for purposes of illustration, various modifications may be made without deviating from the scope of the invention. Accordingly, the invention is not limited except as by the appended claims.

## Claims

1. A process of preparing a beta-lactam, comprising the scheme 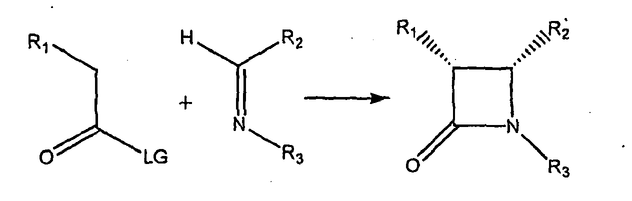 wherein
R₁ is thiol, or protected thiol;
LG is a leaving group;
R₂ is linear, branched or cyclic alkyl, alkenyl, alkynyl, or aryl where R₂ is optionally substituted with one or more of halogen, hydroxyl, alkoxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and
R₃ is hydrogen.

2. The process of claim 1 wherein (R₂)(H)C=N-R₃ is prepared by reaction between an aldehyde of the formula R₂-CHO, and an amine of the formula R₃-NH₂.

3. The process of claim 1 conducted in a chlorinated solvent.

4. The process of claim 1 wherein R₁ is phenyl and R₂ is phenyl.

5. A compound of the formula 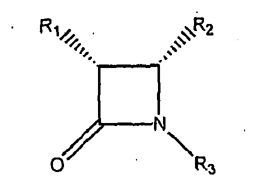 wherein R₁ is thiol (SH), R₂ is phenyl and R₃ is hydrogen.

6. A process for preparing a beta lactam, comprising the scheme 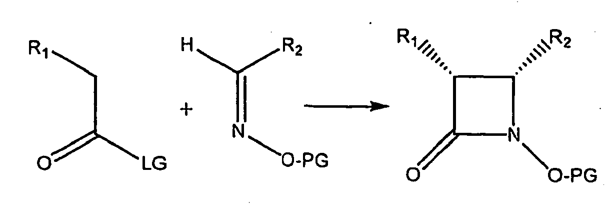 wherein
R₁ is thiol, or protected thiol;
LG is a leaving group;
R₂ is linear, branched or cyclic alkyl, alkenyl, alkynyl or aryl, where R₂ may be optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and
PG is a protecting group.

7. A compound of the formula 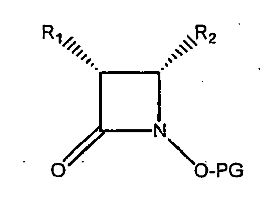 R₁ is thiol, or protected thiol;
R₂ is linear, branched or cyclic alkyl, alkenyl, alkynyl or aryl, where R₂ may be optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and
PG is a protecting group.

8. The compound of claim 7 wherein R₁ is a protected thiol group, and the protecting group is selected from triphenylmethyl (trityl, Trt), acetamidomethyl (Acm), benzamidomethyl, 1-ethoxyethyl and benzoyl.

9. A process of opening a beta-lactam ring, comprising the scheme 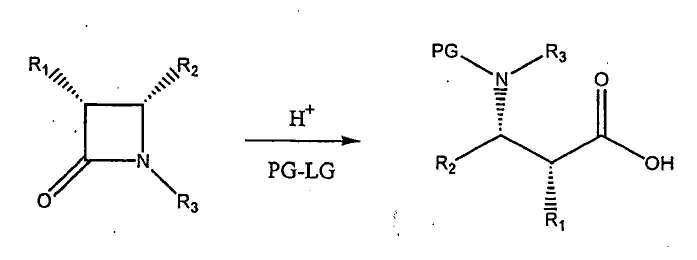 wherein
R₁ is thiol, or protected thiol;
LG is a leaving group;
PG is an amino protecting group;
R₂ is linear, branched or cyclic alkyl, alkenyl, alkynyl, or aryl where R₂ is optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms;
R₃ is hydrogen, C₁-C₅ is linear, branched or cyclic alkyl or aryl where R₃ is optionally substituted with one or more halogens, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and
H⁺ is a proton source.

10. The process of claim 9 wherein the beta-lactam was prepared by the process of claim 1.

11. The process of claim 9 wherein the beta-lactam was prepared by the process of claim 2.

12. The process of claim 9 wherein the ring-opened product is purified by column chromatography followed by recrystallization.

13. The process of claim 12 wherein recrystallization is performed with an organic solvent.

14. The process of claim 9 conducted in a mixture of organic solvent and aqueous acid.

15. The process of claim 9 wherein R₁ is thiophenyl, R₂ is phenyl, and R₃ is hydrogen.

16. An isoserine compound of the formula 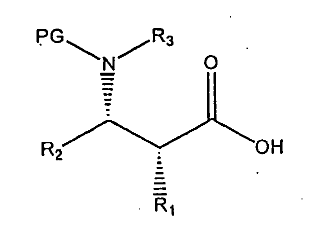 wherein
R₁ is thiol, or protected thiol;
PG is an amino protecting group;
R₂ is linear, branched or cyclic alkyl, alkenyl, alkynyl, or aryl where R₂ is optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms;
R₃ is hydrogen, C₁-C₅ is linear, branched or cyclic alkyl or aryl where R₃ is optionally substituted with one or more halogens, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms;
and salts thereof.

17. An isoserine compound of claim 16 wherein
R₁ is thiol or protected thiol;
R₂ is aryl;
R₃ is hydrogen;
and salts thereof.

18. A compound of the formula 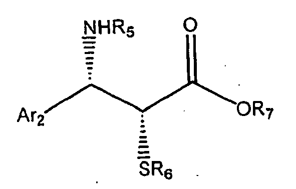 wherein Ar₂ is an aryl group R₅ is selected from hydrogen, benzoyl and tBOC, R₆ is a thiol protecting group, and R₇ is H.

19. The compound of claim 18 wherein the thiol protecting group is triphenylmethyl (trityl, Trt), acetamidomethyl (Acm), benzamidomethyl, 1-ethoxyethyl or benzoyl.

20. A process comprising the scheme 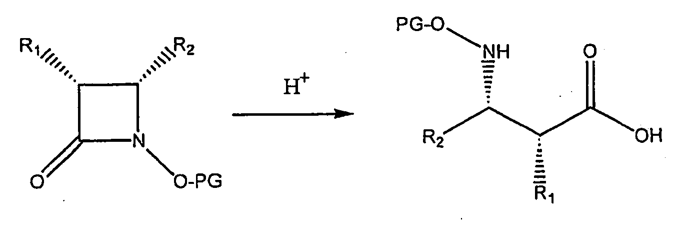 R₁ is thiol, or protected thiol;
R₂ is linear, branched or cyclic alkyl, alkenyl, alkynyl or aryl, where R₂ may be optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms; and
PG is a protecting group.

21. A process of replacing a thioaryl group with a hydroxyl group according to the scheme 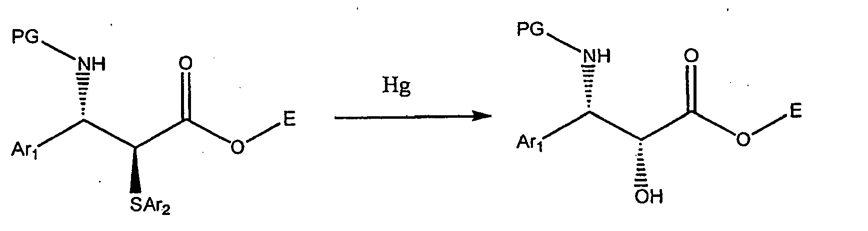 wherein PG is an amine protecting group, Ar₁ arid Ar₂ are aryl groups, E is hydrogen or an organic group, and Hg represents a mercury-containing oxidizing agent.

22. The process of claim 21 wherein PG is benzoyl or tBOC.

23. The process of claim 21 wherein E is hydrogen or C₁-C₆alkyl.

24. The process of claim 21 wherein Ar₁ and Ar₂ are each phenyl.

25. The process of claim 21 wherein Hg is HgO or Hg(CF₃CO₂)₂.

26. A process comprising esterifying a compound of the formula 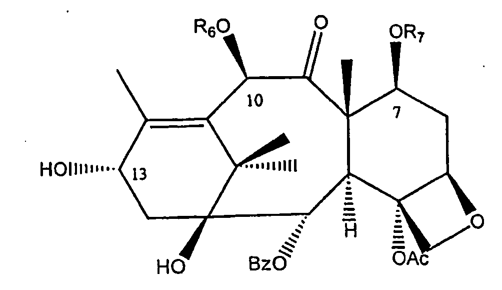 wherein R₆ is acetyl or dichloroacetyl; and R₇ is triethylsilyl, dichloroacetyl or Troc;
with an acid compound of a formula selected from 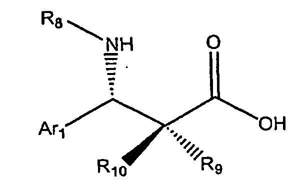 wherein
R₈ is tBOC, PMP, Bz or H;
R₉ is thiophenyl; and
R₁₀ is hydrogen.

27. The process of claim 26 wherein the acid compound has the formula 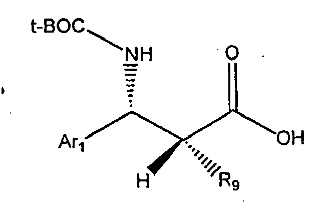 wherein Ar₁ is phenyl and R₉ is thiophenyl.

28. The process of claim 26 wherein the acid compound has the formula 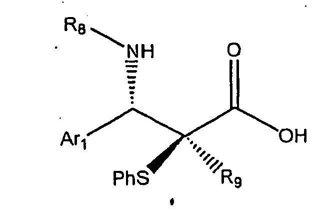 wherein Ar₁ is phenyl, R₈ is hydrogen or PMP, and R₉ is thiophenyl.

29. A compound of the formula 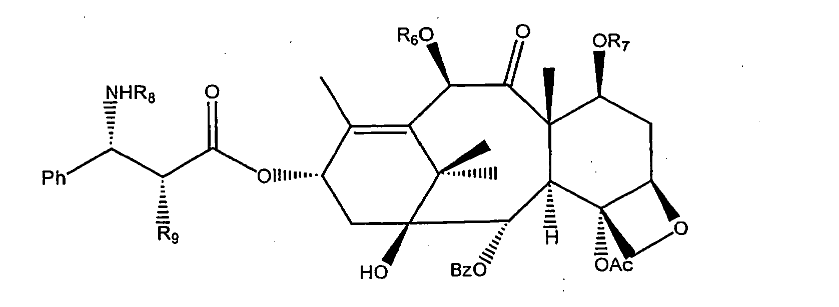 wherein R₆ and R₇ are independently selected from hydrogen, triethylsilyl, acetyl and dichloroacetyl, with the proviso that R₆ and R₇ may not be simultaneously hydrogen, R₈ is tBOC, PMP, Bz or H, and R₉ is thiophenyl.

30. A compound of claim 29 wherein R₆ and R₇ are each dichloroacetyl, R₈ is tBOC and R₉ is thiophenyl.

31. A compound of claim 29 wherein R₆ is acetyl, R₇ is -TES, R₈ is tBOC, and R₉ is thiophenyl.

32. A process comprising the scheme 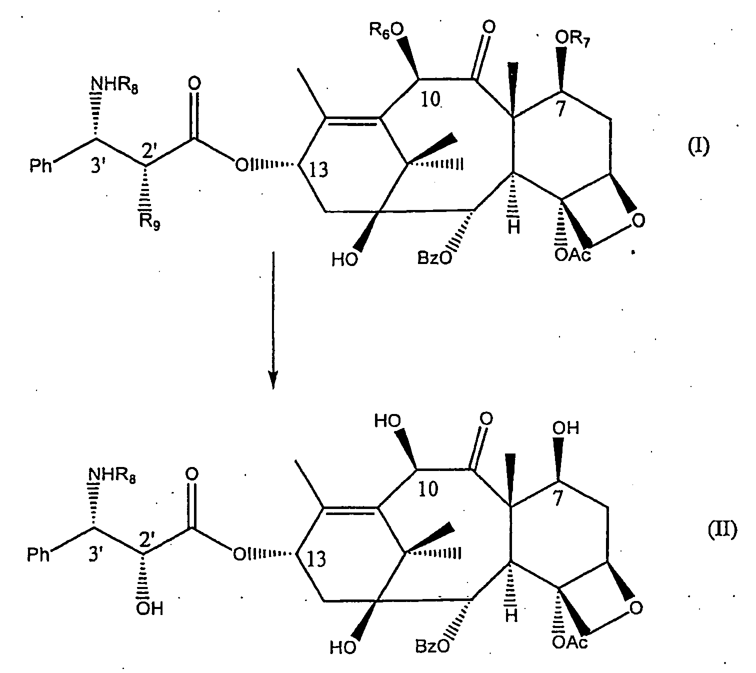 wherein R₆ and R₇ are independently selected from hydrogen, triethylsilyl, acetyl, Troc and dichloroacetyl with the proviso that R₆ and R₇ may not be simultaneously hydrogen, R₈ is tBOC, PMP, Bz or H, and R₉ is triophenyl.

33. The process of claim 32 wherein the compound of structure (I) is deprotected at the 2' position to form an intermediate of structure (Ia), and the intermediate is treated with zinc acetate dehydrate or urea to form the compound of formula (II), where the intermediate has the structure

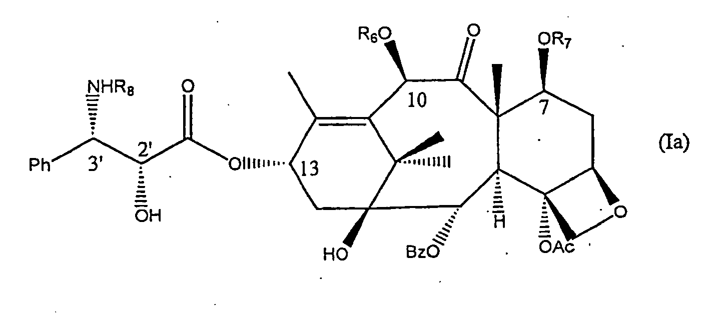

34. The process of claim 32 wherein the compound of formula (I) is treated with protic acid and tertiary amine in an organic solvent to form an intermediate of formula (Ib), and the intermediate is deprotected at the 2' position to form the compound of formula (II), where the intermediate has the structure

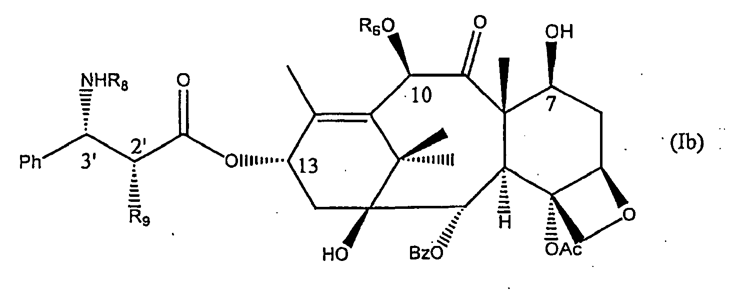

35. A method of preparing TAXOTERE, comprising reacting a compound of structure (III) with tBOC, followed by deprotection of at least one of the 2', 7 and 10 positions, where the compound of structure (III) is 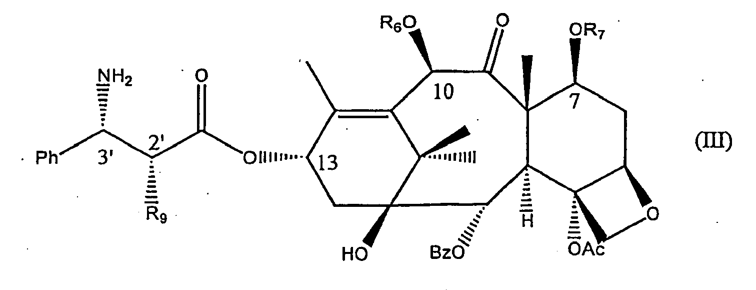 wherein R₆ and R₇ are independently selected from hydrogen, triethylsilyl, acetyl, Troc and dichloroacetyl, with the proviso that R₆ and R₇ may not be simultaneously hydrogen, and R₉ is thiophenyl.

36. A process of coupling a beta lactam to a baccatin III compound according to the following scheme 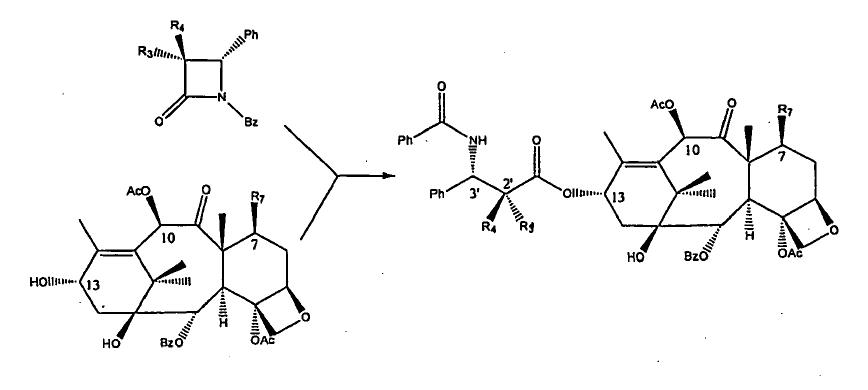 wherein at least one of R₃ or R₄ is either thiol or protected thiol; and the other of R₃ or R₄ is independently selected from hydrogen, hydroxyl, protected hydroxyl, thiol, protected thiol, linear, branched or cyclic alkyl, alkenyl, alkynyl, or aryl where R₁ is optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms;
R₇ is hydroxyl or a protected hydroxyl group; and
the coupling is performed by addition of metal hydride, metal alkoxide or lewis acid to the reaction mixture.

37. The method of claim 36 wherein the coupling is performed by the addition of sodium hydride.

38. The method of claim 36 wherein the coupling is performed by the addition of sodium hexamethyldisilazide.

39. A method for making a compound of formulaes (III) or (IV): 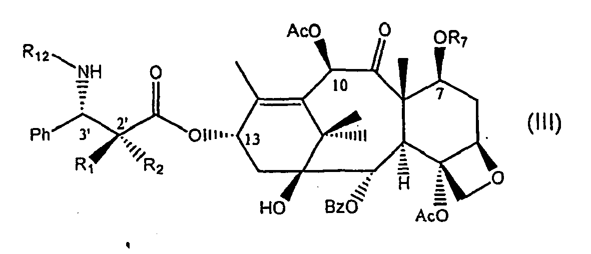 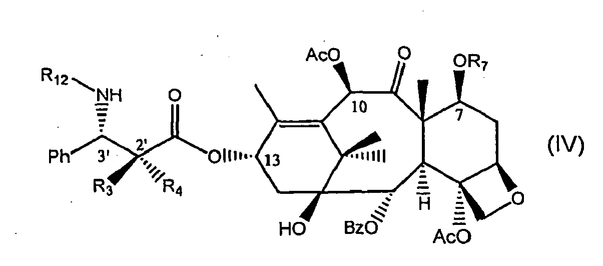 comprising the step of reacting a compound of formula (I) 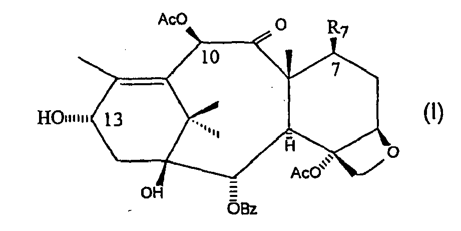 with a compound of formula (IIa) or (IIb) 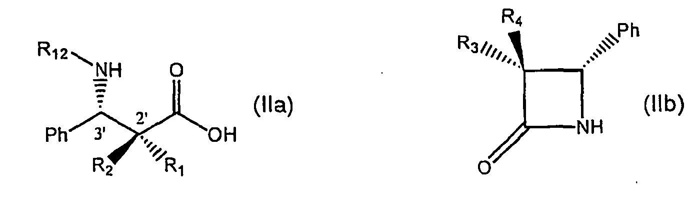 wherein at least one of R₃/R₄ and/or at least one of R₁/R₂ is thiol or protected thiol;
the others of R₁, R₂, R₃ and R₄ being independently selected from hydrogen, hydroxyl, protected hydroxyl, thiol, protected thiol, linear, branched or cyclic alkyl, alkenyl, alkynyl, or aryl where R₃ and R₄ are optionally substituted with one or more of halogen, hydroxyl, alkoxy, aryloxy, heteroaryloxy, amino, alkylamino, dialkylamino, mercapto, alkylthio, arylthio, heteroarylthio, cyano, carboxyl, alkoxycarbonyl where the alkoxy portion contains 1 to 15 carbons, aryloxycarbonyl where the aryloxy portion contains 6 to 20 carbon, or heteroarylcarbonyl where the heteroaryl portion contains 3 to 15 carbon atoms;
R₇ = -COCHCl₂ or triethylsilyl; and
R₁₂ is an amine protecting group.

40. The method of claim 39 wherein the compound of formula (I) is reacted with the compound of formula (IIa).

41. The method of claim 40 wherein R₁₂ is tBOC.

42. The method of claim 41 wherein R₇ is -COCHCl₂.

43. The method of claim 42 wherein R₁ is hydrogen and R₂ is thiophenyl.

44. The method of claim 42 wherein R₁ is OAc and R₂ is thiophenyl.

45. The method of claim 41 wherein R₇ is triethylsilyl.

46. The method of claim 45 wherein R₁ is hydrogen and R₂ is thiophenyl.

47. The method of claim 45 wherein R₁ is OAc and R₂ is thiophenyl.

48. The method of claim 40 wherein R₁₂ is benzoyl.

49. The method of claim 48 wherein R₇ is -COCHCl₂.

50. The method of claim 49 wherein R₁ is hydrogen and R₂ is thiophenyl.

51. The method of claim 49 wherein R₁ is OAc and R₂ is thiophenyl.

52. The method of claim 48 wherein R₇ is triethylsilyl.

53. The method of claim 48 wherein R₁ is hydrogen and R₂ is thiophenyl.

54. The method of claim 52 wherein R₁ is OAc and R₂ is thiophenyl.

55. The method of claim 39 wherein the compound of formula (I) is reacted with the compound of formula (IIb).

56. The method of claim 55 wherein R₁₂ is tBOC.

57. The method of claim 56 wherein R₇ is -COCHCl₂.

58. The method of claim 57 wherein R₃ is -OAc and R₄ is thiophenyl.

59. The method of claim 57 wherein R₃ is -OEE and R₄ is thiophenyl.

60. The method of claim 56 wherein R₇ is triethylsilyl.

61. The method of claim 60 wherein R₃ is -OAc and R₄ is thiophenyl.

62. The method of claim 60 wherein R₃ is -OEE and R₄ is thiophenyl.

63. The method of claim 55 wherein R₁₂ is benzoyl.

64. The method of claim 63 wherein R₇ is -COCHCl₂.

65. The method of claim 64 wherein R₃ is -OAc and R₄ is thiophenyl.

66. The method of claim 64 wherein R₃ is -OEE and R₄ is thiophenyl.

67. The method of claim 63 wherein R₇ is triethylsilyl.

68. The method of claim 67 wherein R₃ is -OAc and R₄ is thiophenyl.

69. The method of claim 67 wherein R₃ is -OEE and R₄ is thiophenyl.

## Patentansprüche

1. Verfahren zur Herstellung eines Beta-Lactams, das folgendes Schema umfasst: 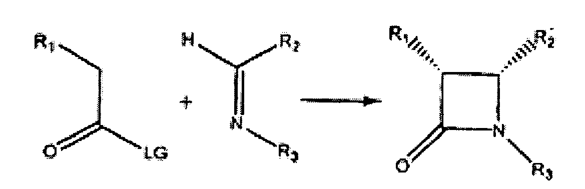 wobei
R₁ Thiol oder geschütztes Thiol ist;
LG eine Abgangsgruppe ist;
R₂ lineares, verzweigtes oder zyklisches Alkyl, Alkenyl, Alkynyl oder Aryl ist,
wobei R₂ optional durch eins oder mehrere von Folgendem ersetzt wird: Halogen, Hydroxyl, Alkoxy, Heteroaryloxy, Amino, Alkylamino, Dialkylamino, Mercapto, Alkylthio, Arylthio, Heteroarylthio, Cyan, Carboxyl, Alkoxycarbonyl, wobei der Alkoxyanteil 1 bis 15 Kohlenstoffatome enthält, Aryloxycarbonyl, wobei der Aryloxyanteil 6 bis 20 Kohlenstoffatome enthält, oder Heteroarylcarbonyl, wobei der Heteroarylanteil 3 bis 15 Kohlenstoffatome enthält; und
R₃ Wasserstoff ist.

2. Verfahren nach Anspruch 1, wobei (R₂)(H)C=N-R₃ durch eine Reaktion zwischen einem Aldehyd der Formel R₂-CHO und einem Amin der Formel R₃-NH₂ hergestellt wird.

3. Verfahren nach Anspruch 1, das in einem chlorierten Lösungsmittel durchgeführt wird.

4. Verfahren nach Anspruch 1, wobei R₁ Phenyl und R₂ Phenyl ist.

5. Verbindung mit folgender Formel: 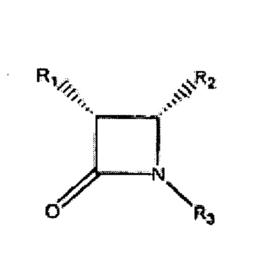 wobei R₁ Thiol (SH), R₂ Phenyl und R₃ Wasserstoff ist.

6. Verfahren zur Herstellung eines Beta-Lactams, das folgendes Schema umfasst: 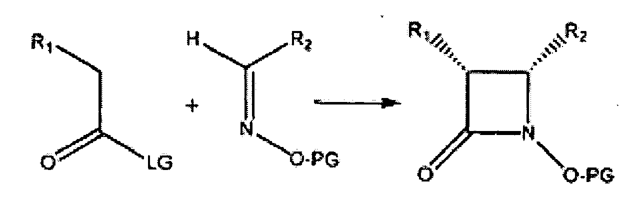 wobei
R₁ Thiol oder geschütztes Thiol ist;
LG eine Abgangsgruppe ist;
R₂ lineares, verzweigtes oder zyklisches Alkyl, Alkenyl, Alkynyl oder Aryl ist,
wobei R₂ optional durch eins oder mehrere von Folgendem ersetzt werden kann: Halogen, Hydroxyl, Alkoxy, Aryloxy, Heteroaryloxy, Amino, Alkylamino, Dialkylamino, Mercapto, Alkylthio, Arylthio, Heteroarylthio, Cyan, Carboxyl, Alkoxycarbonyl, wobei der Alkoxyanteil 1 bis 15 Kohlenstoffatome enthält, Aryloxycarbonyl, wobei der Aryloxyanteil 6 bis 20 Kohlenstoffatome enthält, oder Heteroarylcarbonyl, wobei der Heteroarylanteil 3 bis 15 Kohlenstoffatome enthält; und
PG eine schützende Gruppe ist.

7. Verbindung mit folgender Formel: 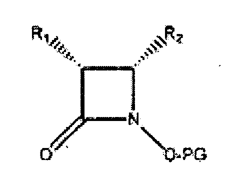 wobei
R₁ Thiol oder geschütztes Thiol ist;
R₂ lineares, verzweigtes oder zyklisches Alkyl, Alkenyl, Alkynyl oder Aryl ist,
wobei R₂ optional durch eines oder mehrere von Folgendem ersetzt werden kann: Halogen, Hydroxyl, Alkoxy, Aryloxy, Heteroaryloxy, Amino, Alkylamino, Dialkylamino, Mercapto, Alkylthio, Arylthio, Heteroarylthio, Cyan, Carboxyl, Alkoxycarbonyl, wobei der Alkoxyanteil 1 bis 15 Kohlenstoffatome enthält, Aryloxycarbonyl, wobei der Aryloxyanteil 6 bis 20 Kohlenstoffatome enthält, oder Heteroarylcarbonyl, wobei der Heteroarylanteil 3 bis 15 Kohlenstoffatome enthält; und
PG eine schützende Gruppe ist.

8. Verbindung nach Anspruch 7, wobei R1 eine geschützte Thiolgruppe ist und die schützende Gruppe aus Triphenylmethyl (Trityl, Trt), Acetamidmethyl (Acm), Benzamidmethyl, 1-Ethoxyethyl und Benzoyl ausgewählt wird.

9. Verfahren zum Öffnen eines Beta-Lactam-Rings, der folgendes Schema umfasst: 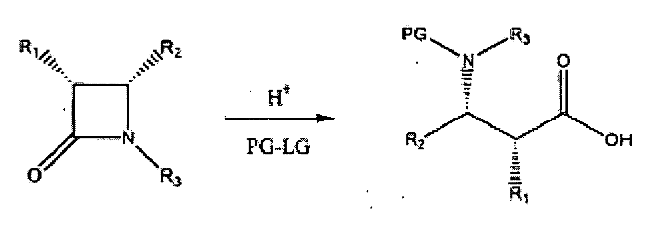 wobei
R₁ Thiol oder geschütztes Thiol ist;
LG eine Abgangsgruppe ist;
PG eine Amino schützende Gruppe ist;
R₂ lineares, verzweigtes oder zyklisches Alkyl, Alkenyl, Alkynyl oder Aryl ist,
wobei R₂ optional durch eins oder mehrere von Folgendem ersetzt wird: Halogen, Hydroxyl, Alkoxy, Aryloxy, Heteroaryloxy, Amino, Alkylamino, Dialkylamino, Mercapto, Alkylthio, Arylthio, Heteroarylthio, Cyan, Carboxyl, Alkoxycarbonyl, wobei der Alkoxyanteil 1 bis 15 Kohlenstoffatome enthält, Aryloxycarbonyl, wobei der Aryloxyanteil 6 bis 20 Kohlenstoffatome enthält, oder Heteroarylcarbonyl, wobei der Heteroarylanteil 3 bis 15 Kohlenstoffatome enthält;
R₃ Wasserstoff ist, C₁-C₅ lineares, verzweigtes oder zyklisches Alkyl oder Aryl ist,
wobei R₃ optional durch eins oder mehrere von Folgendem ersetzt wird: Halogene, Hydroxyl, Alkoxy, Aryloxy Heteroaryloxy, Amino, Alkylamino, Dialkylamino, Mercapto, Alkylthio, Arylthio, Heteroarylthio, Cyan, Carboxyl, Alkoxycarbonyl, wobei der Alkoxyanteil 1 bis 15 Kohlenstoffatome enthält, Aryloxycarbonyl, wobei der Aryloxyanteil 6 bis 20 Kohlenstoffatome enthält, oder Heteroarylcarbonyl, wobei der Heteroarylanteil 3 bis 15 Kohlenstoffatome enthält; und
H⁺ eine Protonenquelle ist.

10. Verfahren nach Anspruch 9, wobei das Beta-Lactam durch das Verfahren nach Anspruch 1 hergestellt wurde.

11. Verfahren nach Anspruch 9, wobei das Beta-Lactam durch das Verfahren nach Anspruch 2 hergestellt wurde.

12. Verfahren nach Anspruch 9, wobei das ringgeöffnete Produkt durch Säulenchromatographie, gefolgt von Rekristallisierung, gereinigt wird.

13. Verfahren nach Anspruch 12, wobei die Rekristallisierung mit einem organischen Lösungsmittel durchgeführt wird.

14. Verfahren nach Anspruch 9, das in einer Mischung aus einem organischen Lösungsmittel und einer wässrigen Säure durchgeführt wird.

15. Verfahren nach Anspruch 9, wobei R₁ Thiophenyl, R₂ Phenyl und R₃ Wasserstoff ist.

16. Isoserinverbindung mit folgender Formel: 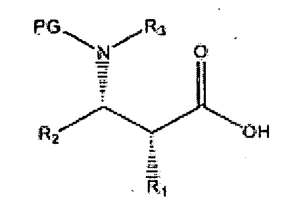 wobei
R₁ Thiol oder geschütztes Thiol ist;
PG eine Amino schützende Gruppe ist;
R₂ lineares, verzweigtes oder zyklisches Alkyl, Alkenyl, Alkynyl oder Aryl ist,
wobei R₂ optional durch eins oder mehrere von Folgendem ersetzt wird: Halogen, Hydroxyl, Alkoxy, Aryloxy, Heteroaryloxy, Amino, Alkylamino, Dialkylamino, Mercapto, Alkylthio, Arylthio, Heteroarylthio, Cyan, Carboxyl, Alkoxycarbonyl, wobei der Alkoxyanteil 1 bis 15 Kohlenstoffatome enthält, Aryloxycarbonyl, wobei der Aryloxyanteil 6 bis 20 Kohlenstoffatome enthält, oder Heteroarylcarbonyl, wobei der Heteroarylanteil 3 bis 15 Kohlenstoffatome enthält;
R₃ Wasserstoff ist, C₁-C₅ lineares, verzweigtes oder zyklisches Alkyl oder Aryl ist,
wobei R₃ optional durch eins oder mehrere von Folgendem ersetzt wird: Halogene, Hydroxyl, Alkoxy, Aryloxy Heteroaryloxy, Amino, Alkylamino, Dialkylamino, Mercapto, Alkylthio, Arylthio, Heteroarylthio, Cyan, Carboxyl, Alkoxycarbonyl, wobei der Alkoxyanteil 1 bis 15 Kohlenstoffatome enthält, Aryloxycarbonyl, wobei der Aryloxyanteil 6 bis 20 Kohlenstoffatome enthält, oder Heteroarylcarbonyl, wobei der Heteroarylanteil 3 bis 15 Kohlenstoffatome enthält;
und Salze davon.

17. Isoserinverbindung nach Anspruch 16, wobei
R₁ Thiol oder geschütztes Thiol ist;
R₂ Aryl ist;
R₃ Wasserstoff ist;
und Salze davon.

18. Verbindung mit der Formel 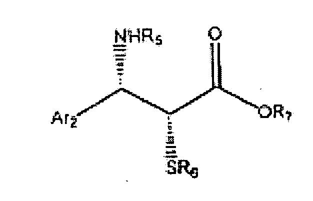 wobei Ar₂ eine Arylgruppe ist, R₅ aus Wasserstoff, Benzoyl und tBOC ausgewählt wird, R₆ eine Thiol schützende Gruppe ist und R₇ H ist.

19. Verbindung nach Anspruch 18, wobei die Thiol schützende Gruppe Triphenylmethyl (Trityl, Trt), Acetamidmethyl (Acm), Benzamidmethyl, 1-Ethoxyethyl oder Benzoyl ist.

20. Verfahren, das Folgendes Schema umfasst: 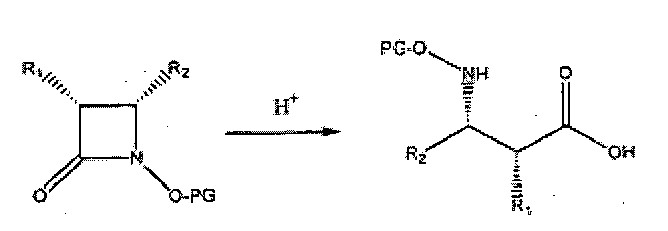 wobei
R₁ Thiol oder geschütztes Thiol ist;
R₂ lineares, verzweigtes oder zyklisches Alkyl, Alkenyl, Alkynyl oder Aryl ist, wobei R₂ optional durch eins oder mehrere von Folgendem ersetzt werden kann: Halogen, Hydroxyl, Alkoxy, Aryloxy, Heteroaryloxy, Amino, Alkylamino, Dialkylamino, Mercapto, Alkylthio, Arylthio, Heteroarylthio, Cyan, Carboxyl, Alkoxycarbonyl, wobei der Alkoxyanteil 1 bis 15 Kohlenstoffatome enthält, Aryloxycarbonyl, wobei der Aryloxyanteil 6 bis 20 Kohlenstoffatome enthält, oder Heteroarylcarbonyl, wobei der Heteroarylanteil 3 bis 15 Kohlenstoffatome enthält; und
PG eine schützende Gruppe ist.

21. Verfahren zum Ersetzen einer Thioarylgruppe durch eine Hydroxylgruppe gemäß dem Schema 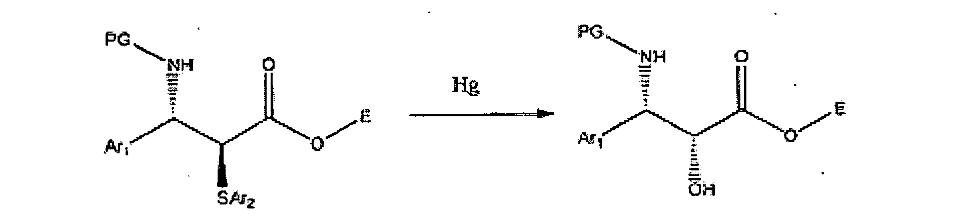 wobei PG eine Amin schützende Gruppe ist, Ar₁ und Ar₂ Arylgruppen sind, E Wasserstoff oder eine organische Gruppe ist und Hg ein Quecksilber haltiges Oxidationsmittel darstellt.

22. Verfahren nach Anspruch 21, wobei PG Benzoyl oder tBOC ist.

23. Verfahren nach Anspruch 21, wobei E Wasserstoff oder C₁-C₆Alkyl ist.

24. Verfahren nach Anspruch 21, wobei Ar₁ und Ar₂ jeweils Phenyl sind.

25. Verfahren nach Anspruch 21, wobei Hg HgO oder Hg(CF₃CO₂)₂ ist.

26. Verfahren, das die Veresterung einer Verbindung mit folgender Formel umfasst: 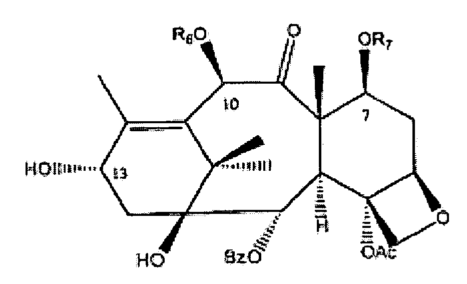 wobei R₆ Acetyl oder Dichloracetyl ist und R₇ Triethylsilyl, Dichloracetyl oder Troc ist;
mit einer Säureverbindung einer Formel, die aus Folgendem ausgewählt wird: 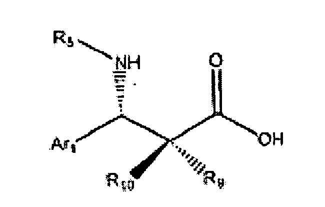 wobei
R₈ tBOC, PMP, Bz oder H ist;
R₉ Thiophenyl ist; und
R₁₀ Wasserstoff ist.

27. Verfahren nach Anspruch 26, wobei die Säureverbindung folgende Formel aufweist: 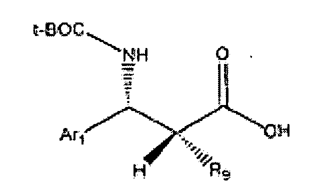 wobei Ar₁ Phenyl und R₉ Thiophenyl ist.

28. Verfahren nach Anspruch 26, wobei die Säureverbindung folgende Formel aufweist: 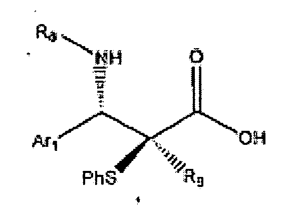 wobei Ar₁ Phenyl, R₈ Wasserstoff oder PMP und R₉ Thiophenyl ist.

29. Verbindung mit der Formel 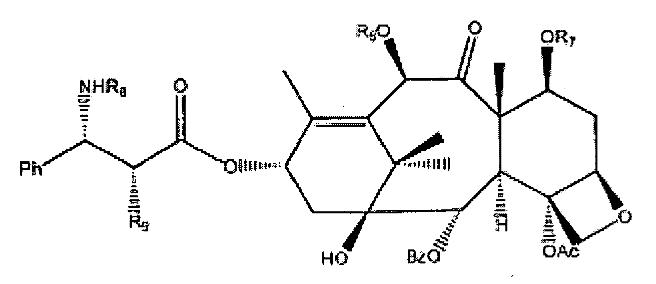 wobei R₆ und R₇ unabhängig aus Folgendem ausgewählt werden: Wasserstoff, Triethylsilyl, Acetyl und Dichloracetyl unter der Bedingung, dass R₆ und R₇ nicht gleichzeitig Wasserstoff sein können, R₈ tBOC, PMP, Bz oder H ist und R₉ Thiophenyl ist.

30. Verbindung nach Anspruch 29, wobei R₆ und R₇ jeweils Dichloracetyl sind, R₈ tBOC ist und R₉ Thiophenyl ist.

31. Verbindung nach Anspruch 29, wobei R₆ Acetyl, R₇ -TES, R₈ tBOC und R₉ Thiophenyl ist.

32. Verfahren, das folgendes Schema umfasst: 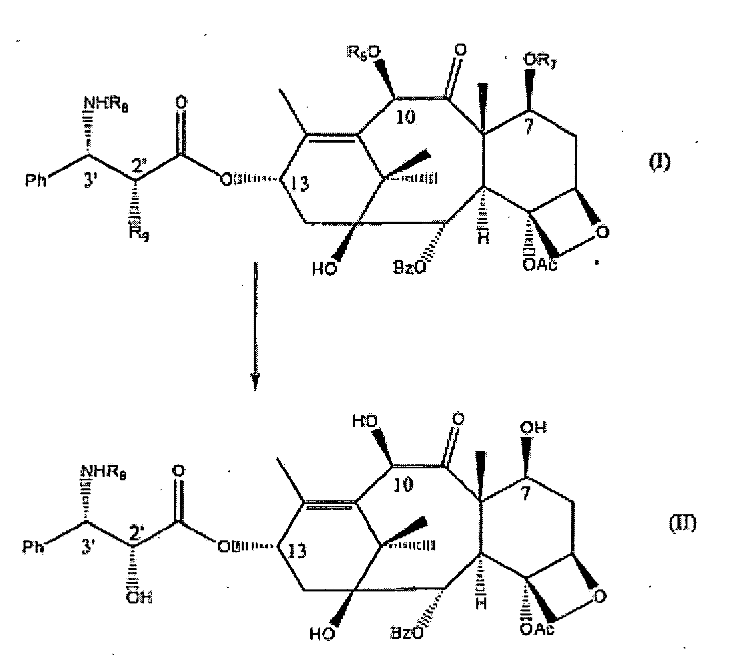 wobei R₆ und R₇ unabhängig aus Folgendem ausgewählt werden: Wasserstoff, Triethylsilyl, Acetyl, Troc und Dichloracetyl unter der Bedingung, dass R₆ und R₇ nicht gleichzeitig Wasserstoff sein können, R₈ tBOC, PMP, Bz oder H ist und R₉ Thiophenyl ist.

33. Verfahren nach Anspruch 32, wobei die Verbindung der Struktur (I) in Position 2' entschützt ist, um ein Zwischenprodukt der Struktur (Ia) zu bilden, und das Zwischenprodukt mit Zinkacetatdehydrat oder Harnstoff behandelt wird, um die Verbindung der Formel (II) zu bilden, wobei das Zwischenprodukt folgende Struktur aufweist:

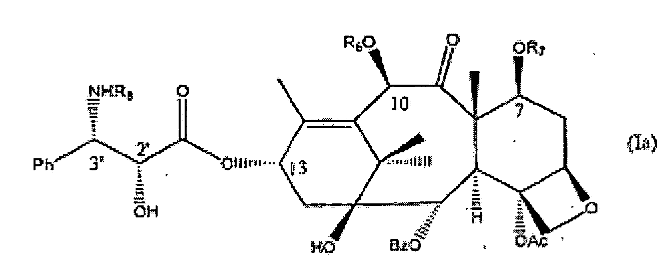

34. Verfahren nach Anspruch 32, wobei die Verbindung der Formel (I) mit protischer Säure und tertiärem Amin in einem organischen Lösungsmittel behandelt wird, um ein Zwischenprodukt der Formel (Ib) zu bilden, und das Zwischenprodukt in Position 2' entschützt wird, um die Verbindung der Formel (II) zu bilden, wobei das Zwischenprodukt folgende Struktur aufweist:

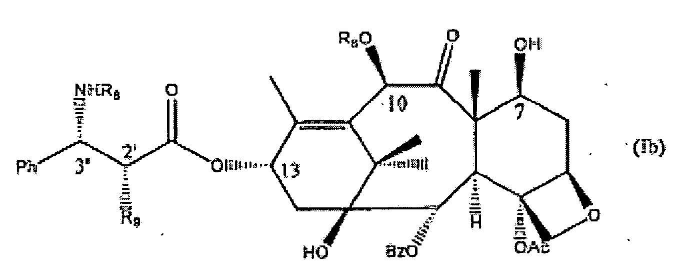

35. Verfahren zur Herstellung von TAXOTERE, das Folgendes umfasst: Reagieren einer Verbindung der Struktur (III) mit tBOC, gefolgt von der Entschützung von mindestens einer der Positionen 2', 7 und 10, wobei die Verbindung der Struktur (III) folgendermaßen lautet: 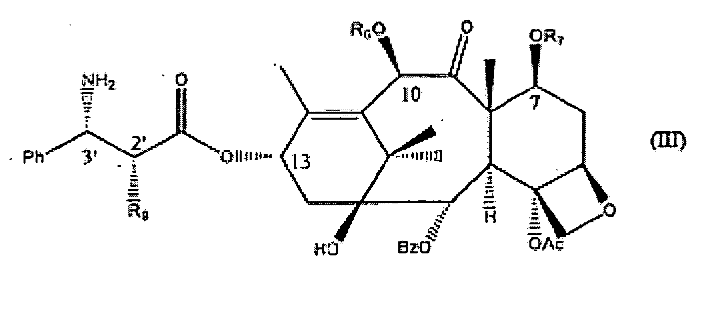 wobei R₆ und R₇ unabhängig aus Folgendem ausgewählt werden: Wasserstoff, Triethylsilyl, Acetyl, Troc und Dichloracetyl unter der Bedingung, dass R₆ und R₇ nicht gleichzeitig Wasserstoff sein können und R₉ Thiophenyl ist.

36. Verfahren zur Kopplung eines Beta-Lactams an eine Baccatin III Verbindung gemäß folgendem Schema: 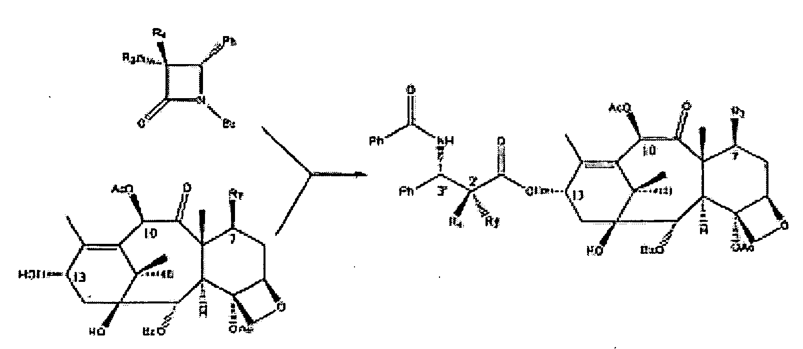 wobei mindestens eines von R₃ oder R₄ entweder Thiol oder geschütztes Thiol ist und das andere von R₃ oder R₄ unabhängig aus Folgendem ausgewählt wird: Wasserstoff, Hydroxyl, geschütztes Hydroxyl, Thiol, geschütztes Thiol, lineares, verzweigtes oder zyklisches Alkyl, Alkenyl, Alkynyl oder Aryl, wobei R₁ optional durch eins oder mehrere von Folgendem ersetzt wird: Halogen, Hydroxyl, Alkoxy, Aryloxy, Heteroaryloxy, Amino, Alkylamino, Dialkylamino, Mercapto, Alkylthio, Arylthio, Heteroarylthio, Cyan, Carboxyl, Alkoxycarbonyl, wobei der Alkoxyanteil 1 bis 15 Kohlenstoffatome enthält, Aryloxycarbonyl, wobei der Aryloxyanteil 6 bis 20 Kohlenstoffatome enthält, oder Heteroarylcarbonyl, wobei der Heteroarylanteil 3 bis 15 Kohlenstoffatome enthält;
R₇ Hydroxyl oder eine geschützte Hydroxylgruppe ist; und
die Kopplung durch Hinzufügung von Metallhydrid, Metallalkoxid oder Lewissäure zur Reaktionsmischung durchgeführt wird.

37. Verfahren nach Anspruch 36, wobei die Kopplung durch Hinzufügung von Natriumhydrid durchgeführt wird.

38. Verfahren nach Anspruch 36, wobei die Kopplung durch Hinzufügung von Natriumhexamethyldisilazid durchgeführt wird.

39. Verfahren zur Herstellung einer Verbindung der Formeln (III) oder (IV): 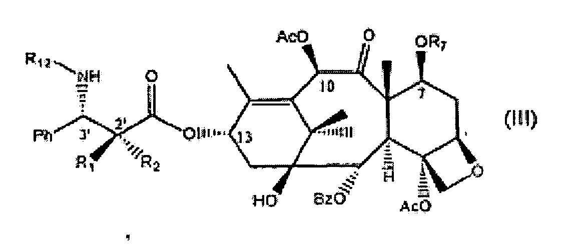 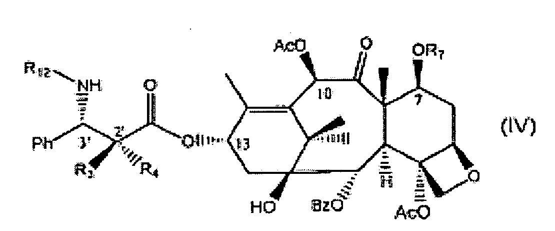 das folgenden Schritt umfasst: Reagieren einer Verbindung der Formel (I) 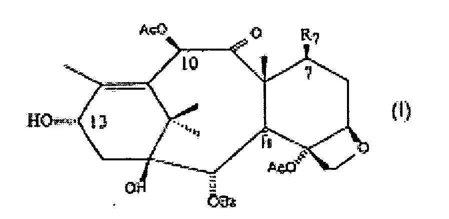 mit einer Verbindung der Formel (IIa) oder (IIb), 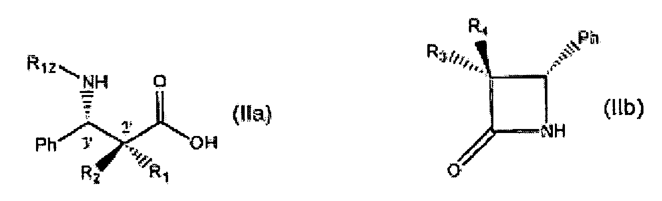 wobei mindestens eines von R₃/R₄ und/oder mindestens eines von R₁/R₂ entweder Thiol oder geschütztes Thiol ist;
die anderen von R₁, R₂, R₃ und R₄ unabhängig aus Folgendem ausgewählt werden: Wasserstoff, Hydroxyl, geschütztes Hydroxyl, Thiol, geschütztes Thiol, lineares, verzweigtes oder zyklisches Alkyl, Alkenyl, Alkynyl oder Aryl, wobei R₃ und R₄ optional durch eins oder mehrere von Folgendem ersetzt werden: Halogen, Hydroxyl, Alkoxy, Aryloxy, Heteroaryloxy, Amino, Alkylamino, Dialkylamino, Mercapto, Alkylthio, Arylthio, Heteroarylthio, Cyan, Carboxyl, Alkoxycarbonyl, wobei der Alkoxyanteil 1 bis 15 Kohlenstoffatome enthält, Aryloxycarbonyl, wobei der Aryloxyanteil 6 bis 20 Kohlenstoffatome enthält, oder Heteroarylcarbonyl, wobei der Heteroarylanteil 3 bis 15 Kohlenstoffatome enthält;
R₇ = -COCHCl₂ oder Triethylsilyl; und
R₁₂ eine Amin schützende Gruppe ist.

40. Verfahren nach Anspruch 39, wobei die Verbindung der Formel (I) mit der Verbindung der Formel (IIa) reagiert wird.

41. Verfahren nach Anspruch 40, wobei R₁₂ tBOC ist.

42. Verfahren nach Anspruch 41, wobei R₇ -COCHCl₂ ist.

43. Verfahren nach Anspruch 42, wobei R₁ Wasserstoff und R₂ Thiophenyl ist.

44. Verfahren nach Anspruch 42, wobei R₁ OAc und R₂ Thiophenyl ist.

45. Verfahren nach Anspruch 41, wobei R₇ Triethylsilyl ist.

46. Verfahren nach Anspruch 45, wobei R₁ Wasserstoff und R₂ Thiophenyl ist.

47. Verfahren nach Anspruch 45, wobei R₁ OAc und R₂ Thiophenyl ist.

48. Verfahren nach Anspruch 40, wobei R₁₂ Benzoyl ist.

49. Verfahren nach Anspruch 48, wobei R₇ -COCHCl₂ ist.

50. Verfahren nach Anspruch 49, wobei R₁ Wasserstoff und R₂ Thiophenyl ist.

51. Verfahren nach Anspruch 49, wobei R₁ OAc und R₂ Thiophenyl ist.

52. Verfahren nach Anspruch 48, wobei R₇ Triethylsilyl ist.

53. Verfahren nach Anspruch 48, wobei R₁ Wasserstoff und R₂ Thiophenyl ist.

54. Verfahren nach Anspruch 52, wobei R₁ OAc und R₂ Thiophenyl ist.

55. Verfahren nach Anspruch 39, wobei die Verbindung der Formel (I) mit der Verbindung der Formel (IIb) reagiert wird.

56. Verfahren nach Anspruch 55, wobei R₁₂ tBOC ist.

57. Verfahren nach Anspruch 56, wobei R₇ -COCHCl₂ ist.

58. Verfahren nach Anspruch 57, wobei R₃ -OAc und R₄ Thiophenyl ist.

59. Verfahren nach Anspruch 57, wobei R₃ -OEE und R₄ Thiophenyl ist.

60. Verfahren nach Anspruch 56, wobei R₇ Triethylsilyl ist.

61. Verfahren nach Anspruch 60, wobei R₃ -OAc und R₄ Thiophenyl ist.

62. Verfahren nach Anspruch 60, wobei R₃ -OEE und R₄ Thiophenyl ist.

63. Verfahren nach Anspruch 55, wobei R₁₂ Benzoyl ist.

64. Verfahren nach Anspruch 63, wobei R₇ -COCHCl₂ ist.

65. Verfahren nach Anspruch 64, wobei R₃ -OAc und R₄ Thiophenyl ist.

66. Verfahren nach Anspruch 64, wobei R₃ -OEE und R₄ Thiophenyl ist.

67. Verfahren nach Anspruch 63, wobei R₇ Triethylsilyl ist.

68. Verfahren nach Anspruch 67, wobei R₃ -OAc und R₄ Thiophenyl ist.

69. Verfahren nach Anspruch 67, wobei R₃ -OEE und R₄ Thiophenyl ist.

## Revendications

1. Procédé de préparation d'une bêta-lactamine, comprenant le schéma 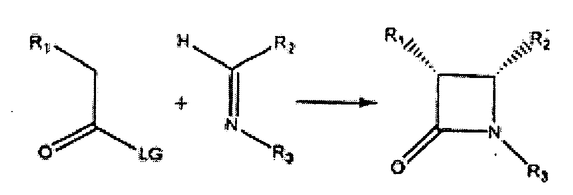 dans lequel
R₁ est un thiol ou un thiol protégé ;
LG est un nucléofuge ;
R₂ est un alkyle, alcényle, alcynyle ou aryle linéaire, ramifié ou cyclique dans lequel R₂ est éventuellement substitué par un ou plusieurs des éléments suivants : halogène, hydroxyle, alkoxy, hétéroaryloxy, amino, alkylamino, dialkylamino, mercapto, alkylthio, arylthio, hétéroarylthio, cyano, carboxyle, alkoxycarbonyle lorsque la partie alkoxy contient entre 1 et 15 atomes de carbone, aryloxycarbonyle lorsque la partie aryloxy contient entre 6 et 20 atomes de carbone ou hétéroarylcarbonyle lorsque la partie hétéroaryle contient entre 3 et 15 atomes de carbone ; et
R₃ est l'hydrogène.

2. Procédé selon la revendication 1, dans lequel (R₂)(H)C=N-R₃ est préparé en faisant réagir un aldéhyde de formule R₂-CHO et une amine de formule R₃-NH₂.

3. Procédé selon la revendication 1 réalisé dans un solvant chloré.

4. Procédé selon la revendication 1, dans lequel R₁ est un phényle et R₂ est un phényle.

5. Composé de formule 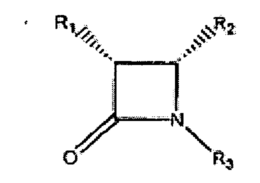 dans lequel R₁ est un thiol (SH), R₂ est un phényle et R₃ est l'hydrogène.

6. Procédé de préparation d'une bêta-lactamine, comprenant le schéma 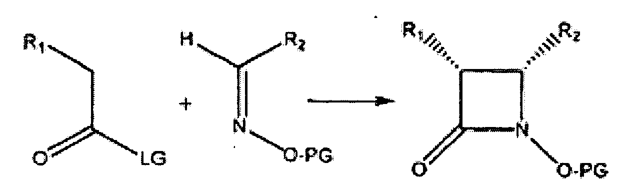 dans lequel
R₁ est un thiol ou un thiol protégé ;
LG est un nucléofuge ;
R₂ est un alkyle, alcényle, alcynyle ou aryle linéaire, ramifié ou cyclique dans lequel R₂ peut être éventuellement substitué par un ou plusieurs des éléments suivants : halogène, hydroxyle, alkoxy, aryloxy, hétéroaryloxy, amino, alkylamino, dialkylamino, mercapto, alkylthio, arylthio, hétéroarylthio, cyano, carboxyle, alkoxycarbonyle lorsque la partie alkoxy contient entre 1 et 15 atomes de carbone, aryloxycarbonyle lorsque la partie aryloxy contient entre 6 et 20 atomes de carbone ou hétéroarylcarbonyle lorsque la partie hétéroaryle contient entre 3 et 15 atomes de carbone ; et
PG est un groupe protecteur.

7. Composé de formule 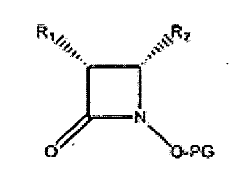 R₁ est un thiol ou un thiol protégé ;
R₂ est un alkyle, alcényle, alcynyle ou aryle linéaire, ramifié ou cyclique dans lequel R₂ peut être éventuellement substitué par un ou plusieurs des éléments suivants : halogène, hydroxyle, alkoxy, aryloxy, hétéroaryloxy, amino, alkylamino, dialkylamino, mercapto, alkylthio, arylthio, hétéroarylthio, cyano, carboxyle, alkoxycarbonyle lorsque la partie alkoxy contient entre 1 et 15 atomes de carbone, aryloxycarbonyle lorsque la partie aryloxy contient entre 6 et 20 atomes de carbone ou hétéroarylcarbonyle lorsque la partie hétéroaryle contient entre 3 et 15 atomes de carbone ; et
PG est un groupe protecteur.

8. Composé selon la revendication 7, dans lequel R₁ est un groupe thiol protégé et le groupe protecteur est sélectionné parmi le triphénylméthyle (trityle, Trt), l'acétamidométhyle (Acm), le benzamidométhyle, le 1-éthoxyéthyle et le benzoyle.

9. Procédé d'ouverture d'un cycle de bêta-lactamine, comprenant le schéma 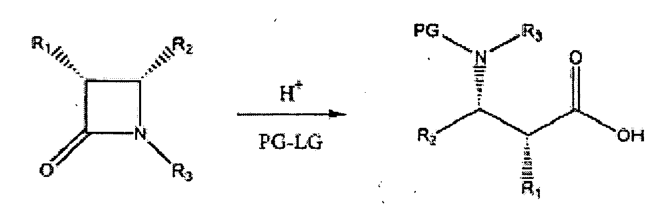 dans lequel
R₁ est un thiol ou un thiol protégé ;
LG est un nucléofuge ;
PG est un groupe protecteur amino ;
R₂ est un alkyle, alcényle, alcynyle ou aryle linéaire, ramifié ou cyclique dans lequel R₂ est éventuellement substitué par un ou plusieurs des éléments suivants : halogène, hydroxyle, alkoxy, aryloxy, hétéroaryloxy, amino, alkylamino, dialkylamino, mercapto, alkylthio, arylthio, hétéroarylthio, cyano, carboxyle, alkoxycarbonyle lorsque la partie alkoxy contient entre 1 et 15 atomes de carbone, aryloxycarbonyle lorsque la partie aryloxy contient entre 6 et 20 atomes de carbone ou hétéroarylcarbonyle lorsque la partie hétéroaryle contient entre 3 et 15 atomes de carbone ;
R₃ est l'hydrogène, C₁-C₅ est un alkyle ou aryle linéaire, ramifié ou cyclique dans lequel R₃ est éventuellement substitué par un ou plusieurs des éléments suivants : halogène, hydroxyle, alkoxy, aryloxy, hétéroaryloxy, amino, alkylamino, dialkylamino, mercapto, alkylthio, arylthio, hétéroarylthio, cyano, carboxyle, alkoxycarbonyle lorsque la partie alkoxy contient entre 1 et 15 atomes de carbone, aryloxycarbonyle lorsque la partie aryloxy contient entre 6 et 20 atomes de carbone ou hétéroarylcarbonyle lorsque la partie hétéroaryle contient entre 3 et 15 atomes de carbone ; et
H⁺ est une source de protons.

10. Procédé selon la revendication 9, dans lequel la bêta-lactamine a été préparée par le procédé de la revendication 1.

11. Procédé selon la revendication 9, dans lequel la bêta-lactamine a été préparée par le procédé de la revendication 2.

12. Procédé selon la revendication 9, dans lequel le produit à cycle ouvert est purifié par chromatographie sur colonne suivie d'une recristallisation.

13. Procédé selon la revendication 12, dans lequel la recristallisation est réalisée à l'aide d'un solvant organique.

14. Procédé selon la revendication 9 réalisé dans un mélange de solvant organique et d'acide aqueux.

15. Procédé selon la revendication 9, dans lequel R₁ est un thiophényle, R₂ est un phényle et R₃ est l'hydrogène.

16. Composé isosérine de formule 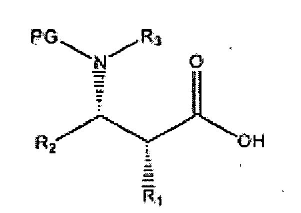 dans lequel
R₁ est un thiol ou un thiol protégé ;
PG est un groupe protecteur amino ;
R₂ est un alkyle, alcényle, alcynyle ou aryle linéaire, ramifié ou cyclique dans lequel R₂ est éventuellement substitué par un ou plusieurs des éléments suivants : halogène, hydroxyle, alkoxy, aryloxy, hétéroaryloxy, amino, alkylamino, dialkylamino, mercapto, alkylthio, arylthio, hétéroarylthio, cyano, carboxyle, alkoxycarbonyle lorsque la partie alkoxy contient entre 1 et 15 atomes de carbone, aryloxycarbonyle lorsque la partie aryloxy contient entre 6 et 20 atomes de carbone ou hétéroarylcarbonyle lorsque la partie hétéroaryle contient entre 3 et 15 atomes de carbone ;
R₃ est l'hydrogène, C₁-C₅ est un alkyle ou aryle linéaire, ramifié ou cyclique dans lequel R₃ est éventuellement substitué par un ou plusieurs des éléments suivants : halogène, hydroxyle, alkoxy, aryloxy, hétéroaryloxy, amino, alkylamino, dialkylamino, mercapto, alkylthio, arylthio, hétéroarylthio, cyano, carboxyle, alkoxycarbonyle lorsque la partie alkoxy contient entre 1 et 15 atomes de carbone, aryloxycarbonyle lorsque la partie aryloxy contient entre 6 et 20 atomes de carbone ou hétéroarylcarbonyle lorsque la partie hétéroaryle contient entre 3 et 15 atomes de carbone ;
et des sels de ceux-ci.

17. Composé isosérine selon la revendication 16, dans lequel
R₁ est un thiol ou un thiol protégé ;
R₂ est un aryle ;
R₃ est l'hydrogène ;
et des sels de ceux-ci.

18. Composé de formule 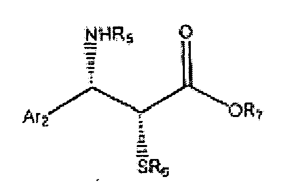 dans lequel Ar₂ est un groupe aryle, R₅ est sélectionné parmi l'hydrogène, le benzoyle et tBOC, R₆ est un groupe protecteur thiol et R₇ est H.

19. Composé selon la revendication 18, dans lequel le groupe protecteur thiol est le triphénylméthyle (trityle, Trt), l'acétamidométhyle (Acm), le benzamidométhyle, le 1-éthoxyéthyle ou le benzoyle.

20. Procédé comprenant le schéma 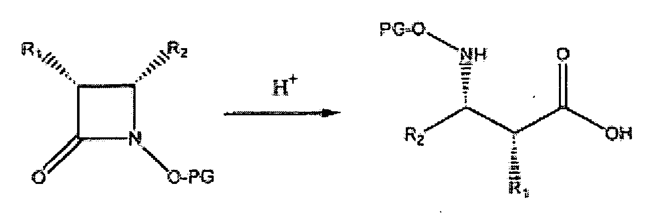 R₁ est un thiol ou un thiol protégé ;
R₂ est un alkyle, alcényle, alcynyle ou aryle linéaire, ramifié ou cyclique dans lequel R₂ peut être éventuellement substitué par un ou plusieurs des éléments suivants : halogène, hydroxyle, alkoxy, aryloxy, hétéroaryloxy, amino, alkylamino, dialkylamino, mercapto, alkylthio, arylthio, hétéroarylthio, cyano, carboxyle, alkoxycarbonyle lorsque la partie alkoxy contient entre 1 et 15 atomes de carbone, aryloxycarbonyle lorsque la partie aryloxy contient entre 6 et 20 atomes de carbone ou hétéroarylcarbonyle lorsque la partie hétéroaryle contient entre 3 et 15 atomes de carbone ; et
PG est un groupe protecteur.

21. Procédé de remplacement d'un groupe thioaryle par un groupe hydroxyle suivant le schéma 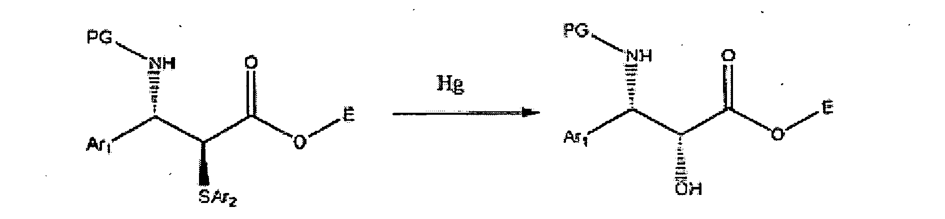 dans lequel PG est un groupe protecteur amine, Ar₁ et Ar₂ sont des groupes aryle, E est l'hydrogène ou un groupe organique et Hg représente un agent oxydant contenant du mercure.

22. Procédé selon la revendication 21, dans lequel PG est le benzoyle ou tBOC.

23. Procédé selon la revendication 21, dans lequel E est l'hydrogène ou un alkyle en C₁-C₆.

24. Procédé selon la revendication 21, dans lequel Ar₁ et Ar₂ sont chacun un phényle.

25. Procédé selon la revendication 21, dans lequel Hg est HgO ou Hg(CF₃CO₂)₂.

26. Procédé comprenant l'estérification d'un composé de formule 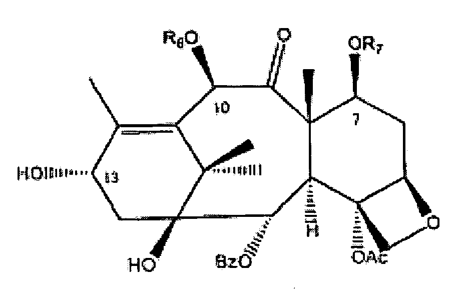 dans lequel R₆ est un acétyle ou dichloroacétyle et R₇ est un triéthylsilyle, dichloroacétyle ou Troc ;
avec un composé acide de formule sélectionnée parmi 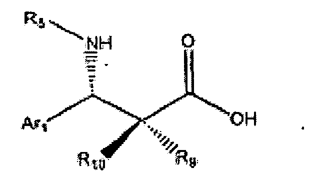 dans lequel
R₈ est tBOC, PMP, Bz ou H ;
R₉ est un thiophényle ; et
R₁₀ est l'hydrogène.

27. Procédé selon la revendication 26, dans lequel le composé acide est de formule 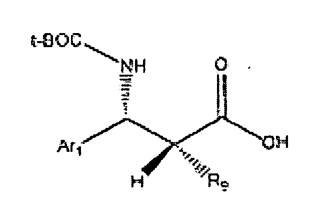 dans lequel Ar₁ est un phényle et R₉ est un thiophényle.

28. Procédé selon la revendication 26, dans lequel le composé acide est de formule 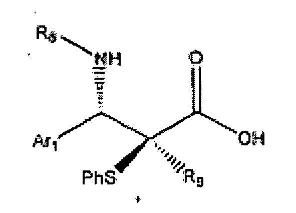 dans lequel Ar₁ est un phényle, R₈ est l'hydrogène ou PMP, et R₉ est un thiophényle.

29. Composé de formule 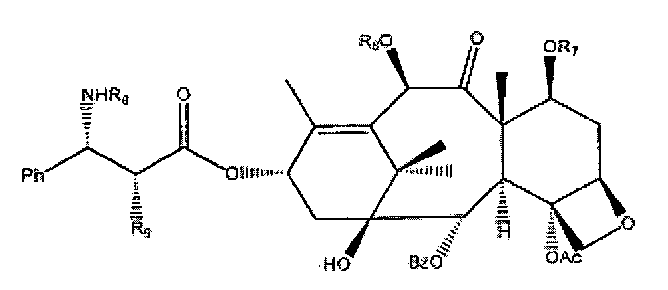 dans lequel R₆ et R₇ sont indépendamment sélectionnés parmi l'hydrogène, triéthylsilyle, acétyle et dichloroacétyle, sous réserve que R₆ et R₇ ne soient pas simultanément l'hydrogène, R₈ est tBOC, PMP, Bz ou H, et R₉ est un thiophényle.

30. Composé selon la revendication 29, dans lequel R₆ et R₇ sont chacun un dichloroacétyle, R₈ est tBOC et R₉ est un thiophényle.

31. Composé selon la revendication 29, dans lequel R₆ est un acétyle, R₇ est -TES, R₈ est tBOC et R₉ est un thiophényle.

32. Procédé comprenant le schéma 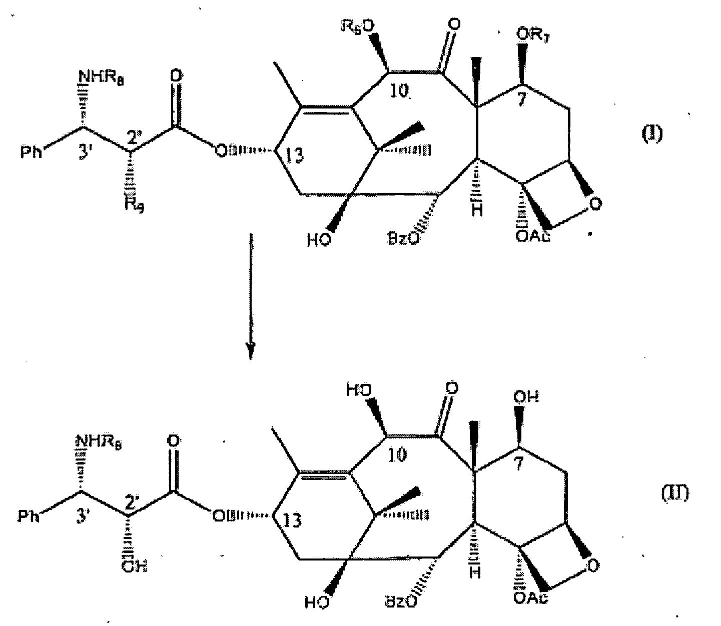 dans lequel R₆ et R₇ sont indépendamment sélectionnés parmi l'hydrogène, triéthylsilyle, acétyle, Troc et dichloroacétyle, sous réserve que R₆ et R₇ ne soient pas simultanément l'hydrogène, R₈ est tBOC, PMP, Bz ou H, et R₉ est un triophényle.

33. Procédé selon la revendication 32, dans lequel le composé de structure (I) est déprotégé au niveau de la position 2' afin de former un intermédiaire de structure (Ia), et l'intermédiaire est traité avec du déshydrate d'acétate de zinc ou de l'urée pour former le composé de formule (II), dans lequel l'intermédiaire présente la structure

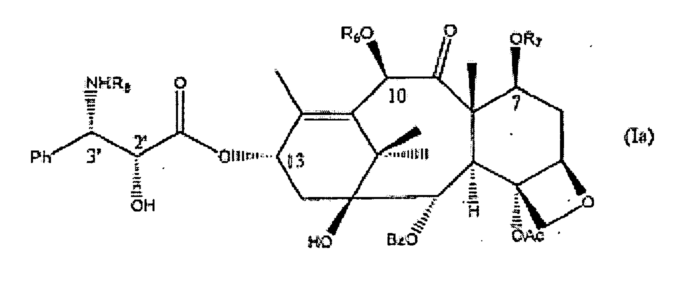

34. Procédé de la revendication 32, dans lequel le composé de formule (I) est traité avec de l'acide protique et une amine tertiaire dans un solvant organique pour former un intermédiaire de formule (Ib), et l'intermédiaire est déprotégé au niveau de la position 2' pour former le composé de formule (II), dans lequel l'intermédiaire présente la structure

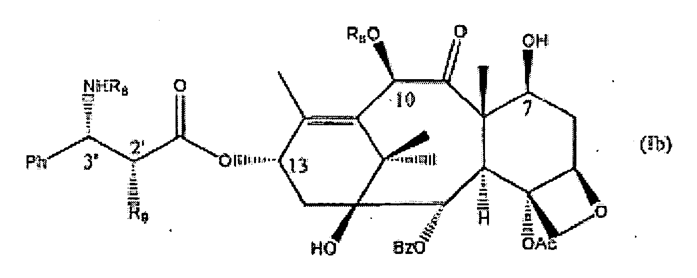

35. Procédé de préparation de TAXOTERE, comprenant la mise en réaction d'un composé de structure (III) avec tBOC, suivie d'une déprotection d'au moins une des positions 2', 7 et 10, dans lequel le composé de structure (III) est 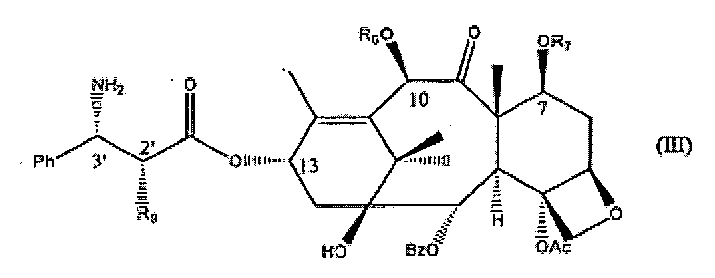 dans lequel R₆ et R₇ sont indépendamment sélectionnés parmi l'hydrogène, triéthylsilyle, acétyle, Troc et dichloroacétyle, sous réserve que R₆ et R₇ ne soient pas simultanément l'hydrogène et R₉ est un thiophényle.

36. Procédé de couplage d'une bêta-lactamine à un composé baccatine III selon le schéma suivant 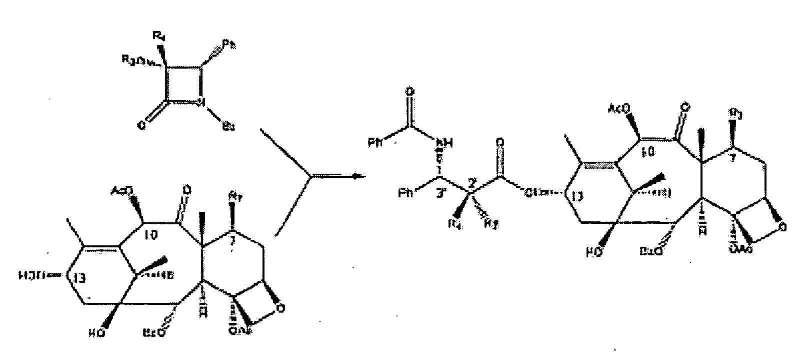 dans lequel au moins l'un de R₃ ou R₄ est un thiol ou un thiol protégé ; et l'autre de R₃ ou R₄ est indépendamment sélectionné parmi l'hydrogène, hydroxyle, hydroxyle protégé, thiol, thiol protégé, alkyle, alcényle, alcynyle ou aryle linéaire, ramifié ou cyclique dans lequel R₁ est éventuellement substitué par un ou plusieurs des éléments suivants : halogène, hydroxyle, alkoxy, aryloxy, hétéroaryloxy, amino, alkylamino, dialkylamino, mercapto, alkylthio, arylthio, hétéroarylthio, cyano, carboxyle, alkoxycarbonyle lorsque la partie alkoxy contient entre 1 et 15 atomes de carbone, aryloxycarbonyle lorsque la partie aryloxy contient entre 6 et 20 atomes de carbone ou hétéroarylcarbonyle lorsque la partie hétéroaryle contient entre 3 et 15 atomes de carbone ;
R₇ est un hydroxyle ou un groupe hydroxyle protégé ; et
le couplage est réalisé par l'ajout d'hydrure métallique, d'alcoolate métallique ou d'acide de Lewis au mélange de réaction.

37. Procédé selon la revendication 36, dans lequel le couplage est réalisé par l'ajout d'hydrure de sodium.

38. Procédé selon la revendication 36, dans lequel le couplage est réalisé par l'ajout d'hexaméthyldisilazide de sodium.

39. Procédé de fabrication d'un composé de formule (III) ou (IV) : 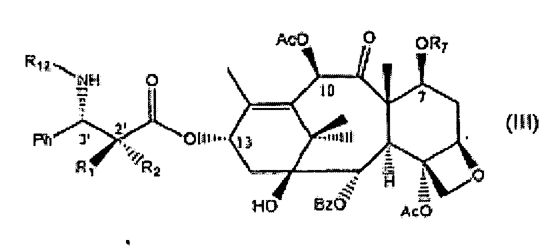 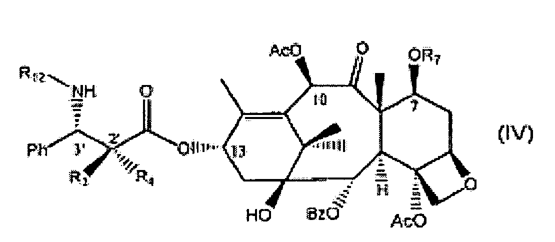 comprenant l'étape de réaction d'un composé de formule (I) 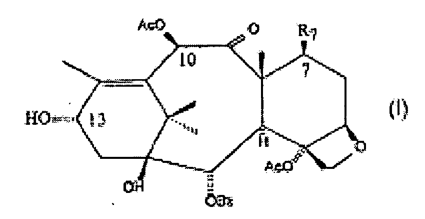 avec un composé de formule (IIa) ou (IIb) 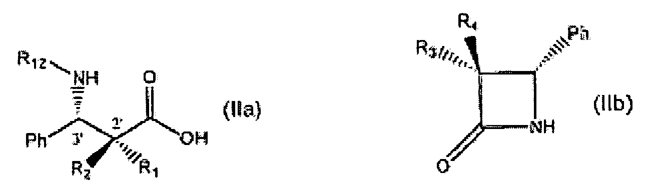 dans lequel au moins l'un de R₃/R₄ et /ou au moins l'un de R₁/R₂ est un thiol ou un thiol protégé ;
les autres parmi R₁, R₂, R₃ et R₄ étant indépendamment sélectionnés parmi l'hydrogène, hydroxyle, hydroxyle protégé, thiol, thiol protégé, alkyle, alcényle, alcynyle ou aryle linéaire, ramifié ou cyclique dans lequel R₃ et R₄ sont éventuellement substitués par un ou plusieurs des éléments suivants : halogène, hydroxyle, alkoxy, aryloxy, hétéroaryloxy, amino, alkylamino, dialkylamino, mercapto, alkylthio, arylthio, hétéroarylthio, cyano, carboxyle, alkoxycarbonyle lorsque la partie alkoxy contient entre 1 et 15 atomes de carbone, aryloxycarbonyle lorsque la partie aryloxy contient entre 6 et 20 atomes de carbone ou hétéroarylcarbonyle lorsque la partie hétéroaryle contient entre 3 et 15 atomes de carbone ;
R₇ = -COCHCl₂ ou triéthylsilyle ; et
R₁₂ est un groupe protecteur amine.

40. Procédé selon la revendication 39, dans lequel le composé de formule (I) est mis en réaction avec le composé de formule (IIa).

41. Procédé selon la revendication 40, dans lequel R₁₂ est tBOC.

42. Procédé selon la revendication 41, dans lequel R₇ est -COCHCl_{2.}

43. Procédé selon la revendication 42, dans lequel R₁ est l'hydrogène et R₂ est un thiophényle.

44. Procédé selon la revendication 42, dans lequel R₁ est OAc et R₂ est un thiophényle.

45. Procédé selon la revendication 41, dans lequel R₇ est un triéthylsilyle.

46. Procédé selon la revendication 45, dans lequel R₁ est l'hydrogène et R₂ est un thiophényle.

47. Procédé selon la revendication 45, dans lequel R₁ est OAc et R₂ est un thiophényle.

48. Procédé selon la revendication 40, dans lequel R₁₂ est un benzoyle.

49. Procédé selon la revendication 48, dans lequel R₇ est -COCHCl₂.

50. Procédé selon la revendication 49, dans lequel R₁ est l'hydrogène et R₂ est un thiophényle.

51. Procédé selon la revendication 49, dans lequel R₁ est OAc et R₂ est un thiophényle.

52. Procédé selon la revendication 48, dans lequel R₇ est un triéthylsilyle.

53. Procédé selon la revendication 48, dans lequel R₁ est l'hydrogène et R₂ est un thiophényle.

54. Procédé selon la revendication 52, dans lequel R₁ est OAc et R₂ est un thiophényle.

55. Procédé selon la revendication 39, dans lequel le composé de formule (I) est mis en réaction avec le composé de formule (IIb).

56. Procédé selon la revendication 55, dans lequel R₁₂ est tBOC.

57. Procédé selon la revendication 56, dans lequel R₇ est -COCHCl₂.

58. Procédé selon la revendication 57, dans lequel R₃ est -OAc et R₄ est un thiophényle.

59. Procédé selon la revendication 57, dans lequel R₃ est -OEE et R₄ est un thiophényle.

60. Procédé selon la revendication 56, dans lequel R₇ est un triéthylsilyle.

61. Procédé selon la revendication 60, dans lequel R₃ est -OAc et R₄ est un thiophényle.

62. Procédé selon la revendication 60, dans lequel R₃ est -OEE et R₄ est un thiophényle.

63. Procédé selon la revendication 55, dans lequel R₁₂ est un benzoyle.

64. Procédé selon la revendication 63, dans lequel R₇ est -COCHCl₂.

65. Procédé selon la revendication 64, dans lequel R₃ est -OAc et R₄ est un thiophényle.

66. Procédé selon la revendication 64, dans lequel R₃ est -OEE et R₄ est un thiophényle.

67. Procédé selon la revendication 63, dans lequel R₇ est un triéthylsilyle.

68. Procédé selon la revendication 67, dans lequel R₃ est -OAc et R₄ est un thiophényle.

69. Procédé selon la revendication 67, dans lequel R₃ est -OEE et R₄ est un thiophényle.
